# EUROPEAN PATENT APPLICATION

(11) **EP 4 850 657 A1**
(43) Date of publication of application: **09.12.2026**
(21) Application number: 24922048.4
(22) Date of filing: 18.12.2024
(51) Int. Cl.: A61K 6/884, A61K 6/16, A61K 6/17, A61K 6/62, A61K 6/71, A61K 6/76, A61K 6/77, A61K 6/887, A61K 6/893, B29C 64/314, B33Y 70/10, C08F 2/44, C08F 2/50, C08F 20/20

(54) **CURABLE COMPOSITION FOR THREE-DIMENSIONAL STEREOLITHOGRAPHY, METHOD FOR PRODUCING SAME, METHOD FOR PRODUCING THREE-DIMENSIONAL STEREOLITHOGRAPHIC PRODUCT, AND METHOD FOR PRODUCING DENTAL RESTORATION ARTICLE**

(30) Priority: 31.01.2024 JP 2024013311
(71) Applicant: Tokuyama Dental Corporation, Tokyo 110-0016 (JP)
(72) Inventor: SAKATA, Eibu, Tokyo 110-0016 (JP); NAKASHIMA, Kei, Tokyo 110-0016 (JP)
(74) Representative: HGF
(86) International application number: PCT/JP2024/044729
(87) International publication number: WO 2025/164130

(57) **Abstract**

Provided are: a curable composition for three-dimensional photofabrication, which contains 100 parts by mass of a polymerizable monomer component (A), 40-400 parts by mass of an inorganic filler (B), 0.01-5 parts by mass of a photopolymerization initiator (C), and 0.01-2.5 parts by mass of an activation light absorbent (D), and in which 10-90 mass% of the inorganic filler (B) is contained as an organic-inorganic composite filler (E), the value of light scattering index Sc serving as an index of the proportion of light scattered laterally when being irradiated with activation light is 10% or less, and the rate of transmission for activation light in a state prior to being optically cured is 1-50%; and a method for producing the curable composition for three-dimensional photofabrication. Also provided are a method for producing a three-dimensional photofabricated product and a method for producing a dental restoration, in both of which said curable composition for three-dimensional photofabrication is used.

## Description

### TECHNICAL FIELD

The present disclosure relates to a curable composition for three-dimensional photofabrication, a method for producing the same, a method for producing a three-dimensional photofabricated product, and a method for producing a dental restoration.

### BACKGROUND ART

A technique for shaping a three-dimensional fabricated product by irradiating a photocurable composition (also referred to as photocurable resin or photocurable resin composition) containing a polymerizable monomer and a photopolymerization initiator with light (activating light) for activating the photopolymerization initiator and curing the photocurable composition is known as a photofabrication method. There are several methods as the photofabrication method, and among these methods, a vat photopolymerization process is widely used because a device is relatively inexpensive and a fabricated product having a smooth surface can be produced with high accuracy.

In the vat photopolymerization process, a three-dimensional object as a production target is generally obtained as follows. First, a height direction of the three-dimensional object is digitized and ordinated based on three-dimensional shape data indicating a shape of the three-dimensional object, and two-dimensional shape data indicating a cross-sectional shape of the three-dimensional object at each ordinated height is generated. Next, a liquid photocurable composition held in a vat is irradiated with activating light at a predetermined position determined in advance based on the two-dimensional shape data, so that the liquid photocurable composition present at the position is selectively primarily cured to form a fabricated layer having the cross-sectional shape, and the fabricated layer having the cross-sectional shape at each height according to an order of ordinating is sequentially formed and stacked to obtain a stack having a shape corresponding to the shape of the three-dimensional object. Next, the stack is washed with an organic solvent as necessary, and then secondarily cured to obtain a target product.

In the dental field, dental restorations such as dentures and crown prostheses need to be produced highly precisely such that the dental restorations have a unique shape in accordance with the situation in the oral cavity of each individual patient. For this reason, it has been studied to produce a dental restoration by photofabrication according to a vat photopolymerization process based on computer aided design (CAD) data designed by using digital data obtained by oral scanning or the like.

The dental prostheses used in the oral cavity are required not only to have high dimensional (shape) accuracy as described above, but also to have high mechanical strength that can sufficiently withstand a load applied during chewing. In this regard, when an inorganic filler is blended in the photocurable composition, polymerization shrinkage, which is one of causes of a decrease in accuracy, can be reduced, and the mechanical strength and surface hardness of a cured product can be improved. Therefore, it is considered that a vat photopolymerization process by using a photocurable composition blended with an inorganic filler is suitable for photofabrication of dental prostheses, and a curable composition for three-dimensional photofabrication containing such an inorganic filler is also proposed.

For example, Patent Document 1 discloses a composition containing a polymerizable monomer (a), an ultraviolet light absorbing inorganic particle (b), and a photopolymerization initiator (c), as a composition for optical fabrication, which is excellent in shaping accuracy, mechanical characteristics, and transparency, and is particularly suitable for a dental material. Patent Document 2 discloses a composition containing a transparent resin and two or more types of transparent particles having different refractive indexes and Abbe numbers, as a resin composition capable of producing a cured product (three-dimensional fabricated product) having excellent designability. Patent Document 3 discloses as a resin composition for photofabrication capable of obtaining a three-dimensional fabricated product having high strength, high elasticity, and excellent wear resistance, a composition containing a urethanated (meth)acrylic compound (a), a (meth)acrylamide compound (b), a photopolymerization initiator (c), and spherical inorganic particles (d) having an average particle diameter of 0.75 µm to 10 µm, in which a content of the spherical inorganic particles (d) is 50 parts by mass to 400 parts by mass with respect to 100 parts by mass of the total amount of the polymerizable monomers. In addition, Patent Document 4 discloses that an organic-inorganic composite filler is added to a dental curable composition, the organic-inorganic composite filler including inorganic aggregated particles in which inorganic primary particles having an average particle diameter of 10 nm to 1,000 nm are aggregated, and an organic resin phase including a water absorbent resin and a polymerized cured product of a polymerizable monomer that covers a surface of each inorganic primary particle and bonds each inorganic primary particle to each other, for the purpose of reducing viscosity of a paste, improving operability, preventing polymerization shrinkage at the time of curing, and improving mechanical strength of a cured product while maintaining a surface smoothness of the cured product.

### Citation List

### Patent Documents

Patent Document 1: PCT International Publication No. WO2018/074380
Patent Document 2: Japanese Unexamined Patent Application Publication No. 2020-180171
Patent Document 3: Japanese Unexamined Patent Application Publication No. 2022-41276
Patent Document 4: PCT International Publication No. WO2013/039169
Patent Document 5: Japanese Patent No. 3917204

### DISCLOSURE OF THE INVENTION

### Problems to be Solved by the Invention

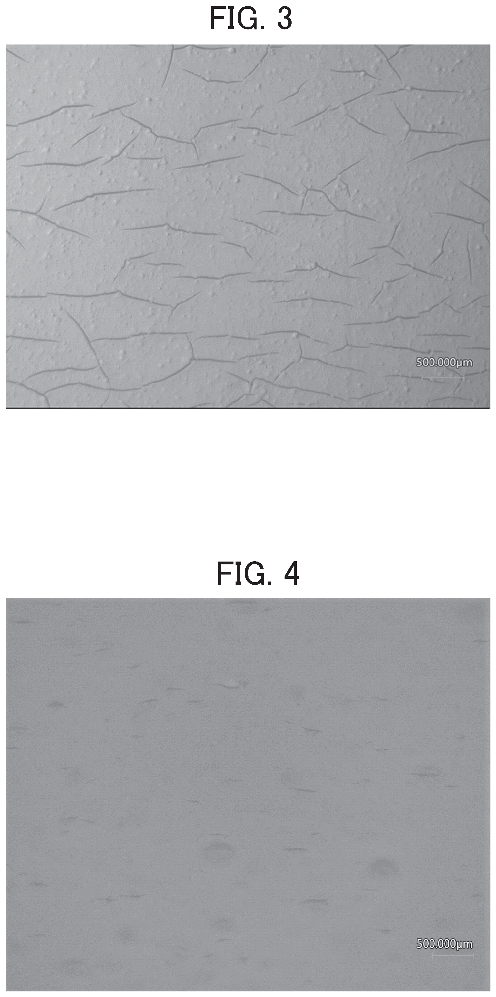

In Patent Documents 1 to 3, since a composition in which an inorganic filler is added to a polymerizable monomer is used, it is possible to obtain a fabricated product having good mechanical strength, elastic modulus, and wear resistance. However, since the composition for optical fabrication described in Patent Document 1 has low fluidity, the shape of an applicable fabricated product may be limited. In addition, in the resin composition described in Patent Document 2, when the resin composition is left to stand for a long period of time, light-transmitting particles (glass filler or the like) contained in the composition may settle. Further, in the resin composition for photofabrication described in Patent Document 3, it has been revealed that fine cracks which are difficult to visually distinguish are often generated on a surface of the fabricated product (see Fig. 4). When such a fabricated product having fine cracks is used as a dental prosthesis, there is a possibility that the dental prosthesis may become a starting point for fracture in the oral cavity, which is a problem.

Therefore, an object of the present disclosure is to provide a technique capable of producing a three-dimensional fabricated product with high accuracy and high strength without generating cracks on a surface at the time of producing the three-dimensional fabricated product by photofabrication by a vat photopolymerization process using a curable composition for three-dimensional photofabrication in which a certain amount of an inorganic filler is blended for high strength.

### Means for Solving the Problems

A first aspect of the present disclosure is a curable composition for three-dimensional photofabrication to be used as a liquid photocurable composition held in a vat in a vat photopolymerization process for producing a three-dimensional photofabricated product by irradiating a predetermined position of the liquid photocurable composition with activating light (hereinafter, also referred to as "specific activating light") including light having a specific wavelength λ (nm) in an ultraviolet light range or a visible light range and selectively curing the liquid photocurable composition present at the position, the curable composition containing:
100 parts by mass of a polymerizable monomer component (A); 40 parts by mass to 400 parts by mass of an inorganic filler (B) containing a single type or a plurality of types of inorganic particulate matters; 0.01 parts by mass to 5 parts by mass of a photopolymerization initiator (C) having a function of initiating photopolymerization by irradiation with the specific activating light; and 0.01 parts by mass to 2.5 parts by mass of an activating light absorbent (D) having a function of absorbing the specific activating light and having no photopolymerization initiation ability,
80% or more of all primary particles constituting the inorganic filler (B) being particles having a particle diameter of 0.05 µm to 5.0 µm in a particle size distribution of the inorganic filler (B) measured by a microscopy method using a scanning microscope,
10 mass% to 90 mass% of the inorganic filler (B) being contained as an organic-inorganic composite filler (E) containing particles formed of a composite material of the inorganic filler (B) and a resin (R),
when, among the inorganic filler (B), an inorganic filler not combined with the resin (R) is defined as a non-composite inorganic filler (B1) and an inorganic filler contained as the organic-inorganic composite filler (E) is defined as a composite inorganic filler (B2), 80% or more of all primary particles constituting each of the non-composite inorganic filler (B1) and the composite inorganic filler (B2) being particles having a particle diameter of 0.05 µm to 5.0 µm, the polymerizable monomer component (A), the non-composite inorganic filler (B1), and the organic-inorganic composite filler (E) being such that a value of a light scattering index Sc (%) of a base composition is 10 (%) or less,
the base composition being a composition consisting of the polymerizable monomer component (A), the non-composite inorganic filler (B1), and the organic-inorganic composite filler (E) and having a composition ratio of the components same as a composition ratio of the curable composition for three-dimensional photofabrication,
the light scattering index Sc (%) being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength λ (nm), including light having a wavelength in a range of λ ± 50 (nm) as a main component, and having a maximum intensity in the range, $Sc = \left\{\left({I}_{70}+{I}_{75}+{I}_{80}\right)/\left({I}_{0}\times 3\right)\right\} \times 100$
wherein I₀, I₇₀, I₇₅, and I₈₀ indicate intensities of the transmitted light in directions of emission angles of 0°, 70°, 75°, and 80°, respectively,
a transmittance to the specific activating light measured for a sample having a thickness of 0.5 mm and containing the curable composition for three-dimensional photofabrication being 1% to 50%.

A second aspect of the present disclosure is a method for producing a curable composition for three-dimensional photofabrication according to the present disclosure, the method including:
a mixing step of mixing the polymerizable monomer component (A), the non-composite inorganic filler (B1), the organic-inorganic composite filler (E), the photopolymerization initiator (C), and the activating light absorbent (D), in which
when a refractive index with respect to a line D at 25°C and a refractive index with respect to the specific activating light at 25°C of the polymerizable monomer component (A) are defined as n_{(D)M} and n_{(AL)M}, respectively, a refractive index with respect to the line D at 25°C and a refractive index with respect to the specific activating light at 25°C of the inorganic particulate matters constituting the inorganic filler (B) are defined as n_{(D)F} and n_{(AL)F}, respectively, a refractive index with respect to the specific activating light at 25°C of the resin (R) contained in the organic-inorganic composite filler (E) is defined as n_{(AL)R}, and a composition consisting of the polymerizable monomer component (A), the non-composite inorganic filler (B1), and the organic-inorganic composite filler (E) and having a composition ratio of the components same as a composition ratio of the curable composition for three-dimensional photofabrication serving as a production target is used as a base composition,
in the mixing step, those found to satisfy all of the following conditions 1 to 6 are used as the polymerizable monomer component (A), the non-composite inorganic filler (B1), and the organic-inorganic composite filler (E), condition 1: when a particle diameter of each primary particle constituting the inorganic filler (B) is defined as x (nm) and a circumference ratio is defined as π, a total number of particles having a particle diameter x (nm) within a range of 0.7λ/π to 4λ/π (nm) is 40% or more of the number of all primary particles constituting the inorganic filler (B) in a particle size distribution of the inorganic filler (B) measured by a microscopy method using a scanning microscope; condition 2: when an inorganic particulate matter consisting of an aggregate of a single type of inorganic particles having the refractive index n_{(D)F} in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), each of the non-composite inorganic filler (B1) and the composite inorganic filler (B2)
   (1) contains a single type of the specific inorganic particulate matter (b1),
   (2) contains a plurality of types of the specific inorganic particulate matters (b1), at least one of the plurality of types of specific inorganic particulate matters (b1) occupying 10 mass% or more of a total mass of each of the non-composite inorganic filler (B1) and the composite inorganic filler (B2), or
   (3) contains 90 mass% or more and less than 100 mass% of a single type or a plurality of types of the specific inorganic particulate matters (b1) and more than 0 mass% and 10 mass% or less of a single type or a plurality of types of the non-specific inorganic particulate matters (b2), at least one of the single type or the plurality of types of specific inorganic particulate matters (b1) occupying 10 mass% or more of a total mass of each of the non-composite inorganic filler (B1) and the composite inorganic filler (B2);
condition 3: the refractive index n_{(D)M} is within a range of 1.490 to 1.550;
condition 4: when a refractive index at which a difference with the refractive index n_{(D)M} is largest among the refractive indexes n_{(D)F} of at least one of the specific inorganic particulate matters (b1) occupying 10 mass% or more of the total mass of each of the non-composite inorganic filler (B1) and the composite inorganic filler (B2) is defined as n_{(D)Fh}, an absolute value |n_{(D)Fh} - n_{(D)M}| of the difference between n_{(D)Fh} and n_{(D)M} is 0.035 or less;
condition 5: the refractive index n_{(AL)F} of at least one of the inorganic particulate matters (b1) occupying 10 mass% or more of the total mass of each of the non-composite inorganic filler (B1) and the composite inorganic filler (B2), the refractive index n_{(AL)M}, and the refractive index n_{(AL)R} satisfy all relations shown in the following formulae (i) to (iii), $- 0.015 < \left({n}_{\left(AL\right) F}-{n}_{\left(AL\right) M}\right) < 0.025$ (i), $- 0.015 < \left({n}_{\left(AL\right) R}-{n}_{\left(AL\right) M}\right) < 0.030$ (ii), and $- 0.020 < \left({n}_{\left(AL\right) F}-{n}_{\left(AL\right) R}\right) < 0.020$ (iii); and
condition 6: a value of a light scattering index Sc (%) is 10% or less, the light scattering index Sc (%) being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength λ (nm), including light having a wavelength in a range of λ ± 50 (nm) as a main component, and having a maximum intensity in the range, $Sc = \left\{\left({I}_{70}+{I}_{75}+{I}_{80}\right)/\left({I}_{0}\times 3\right)\right\} \times 100$
wherein I₀, I₇₀, I₇₅, and I₈₀ indicate intensities of the transmitted light in directions of emission angles of 0°, 70°, 75°, and 80°, respectively.

A third aspect of the present disclosure is a method for producing a three-dimensional photofabricated product, which is a method for producing a three-dimensional photofabricated product by irradiating a predetermined position of a liquid photocurable composition held in a vat with specific activating light and selectively curing the liquid photocurable composition present at the position, the method including:
a shaping step of digitizing and ordinating a height direction of a three-dimensional object based on three-dimensional shape data indicating a shape of the three-dimensional object, generating two-dimensional shape data indicating a cross-sectional shape of the three-dimensional object at each ordinated height, irradiating the liquid photocurable composition held in the vat with the specific activating light at a predetermined position determined in advance based on the two-dimensional shape data, thereby selectively primarily curing the liquid photocurable composition present at the position, forming a fabricated layer having the cross-sectional shape, and sequentially forming and stacking the fabricated layer having the cross-sectional shape at each height according to an order of the ordinating, to obtain a stack having a shape corresponding to the shape of the three-dimensional object;
a washing step of washing, with an organic solvent, the stack obtained in the shaping step; and
a secondary curing step of secondarily curing the stack washed in the washing step by performing additional activating light irradiation or a heating treatment, or both of the additional activating light irradiation and the heating treatment,
the curable composition for three-dimensional photofabrication according to the present disclosure being used as the liquid photocurable composition.

A fourth aspect of the present disclosure is a method for producing a dental restoration, including: producing the dental restoration by the method for producing a three-dimensional photofabricated product according to the present disclosure.

### Effects of the Invention

The curable composition for three-dimensional photofabrication of the present disclosure has fluidity sufficient for shaping with a three-dimensional photofabrication device while containing a certain amount of an inorganic filler for high strength. According to the method for producing a three-dimensional photofabricated product of the present disclosure using the curable composition for three-dimensional photofabrication, it is possible to produce a three-dimensional photofabricated product having excellent mechanical strength and good shape accuracy while suppressing generation of cracks on a surface.

### BRIEF DESCRIPTION OF THE DRAWINGS

Fig. 1 is a view illustrating an optical microscope image (magnification of 50X) of a surface (evaluated as A0) of a three-dimensional photofabricated product obtained in Reference Example 7;
Fig. 2 is a view illustrating an optical microscope image (magnification of 50X) of a surface (evaluated as B) of a three-dimensional photofabricated product obtained in Reference Example 11;
Fig. 3 is a view illustrating an optical microscope image (magnification of 50X) of a surface (evaluated as D) of a three-dimensional photofabricated product obtained in Reference Comparative Example 1;
Fig. 4 is a view illustrating an optical microscope image (magnification of 50X) of a surface (evaluated as C) of a three-dimensional photofabricated product obtained in Reference Comparative Example 9;
Fig. 5 is a graph illustrating a wavelength distribution of measurement light used for measuring a light scattering index Sc in examples and reference examples; and
Fig. 6 is a view illustrating an optical microscope image (magnification of 50X) of a surface (evaluated as A1) of a three-dimensional photofabricated product obtained in Example 1.

### PREFERRED MODE FOR CARRYING OUT THE INVENTION

### 1. Overview of Curable Composition for Three-dimensional Photofabrication of Present Disclosure

A curable composition for three-dimensional photofabrication of the present disclosure (hereinafter, also simply referred to as "curable composition for photofabrication") is a composition to be used as a liquid photocurable composition at the time of producing a three-dimensional object using a vat photopolymerization process, that is, a curable composition for three-dimensional photofabrication by a vat photopolymerization process.

Here, the vat photopolymerization process means a method including a step (hereinafter, also referred to "shaping step") of digitizing and ordinating a height direction of a three-dimensional object based on three-dimensional shape data indicating a shape of the three-dimensional object, generating two-dimensional shape data indicating a cross-sectional shape of the three-dimensional object at each ordinated height, irradiating a liquid photocurable composition held in a vat with activating light at a predetermined position determined in advance based on the two-dimensional shape data, so that the liquid photocurable composition present at the position is selectively (primarily) cured to form a fabricated layer having the cross-sectional shape, and sequentially forming and stacking the fabricated layer having the cross-sectional shape at each height according to an order of ordinating to obtain a stack having a shape corresponding to the shape of the three-dimensional object, and as necessary, performing a washing treatment using an organic solvent (hereinafter, step of performing such treatment is also referred to as "washing step") or a secondary curing treatment (hereinafter, step of performing such treatment is also referred to as "secondary curing step") to obtain a three-dimensional photofabricated product having a shape corresponding to the shape of the three-dimensional object.

As described above, in a photocurable composition in which a certain amount of an inorganic filler is blended in order to improve mechanical strength and surface hardness of a cured product, there may be a problem of a decrease in fluidity or particle sedimentation during storage. In addition, when a fabricated product is produced by a vat photopolymerization process using the resin composition for photofabrication described in Patent Document 3 (although not recognized in Patent Document 3), fine cracks which are difficult to visually distinguish are often generated on the surface of the fabricated product.

The reason why cracks are generated is unclear, but the present inventors have found that cracks are generated when the uncured curable composition adhered to the surface of the stack is washed with an organic solvent after the stack is obtained using a photofabrication device. In addition, since the inorganic filler contained in the curable composition for photofabrication contains a large amount of particles having a particle diameter that causes scattering (specifically, Mie scattering or Rayleigh scattering) when irradiated with the activating light, it is considered that generation of weak activating light (side scattered light) scattered from an optical axis at an irradiation spot causes a very shallow curing depth, and formation of a region (hereinafter, also referred to as "interlayer low-crosslinking-density region") where a crosslinking density is relatively small between layers of the stack also contributes to generation of cracks. That is, permeation of the organic solvent into the interlayer low-crosslinking-density region during washing causes swelling of the region, so that an intermolecular space of a high molecular chain constituting a shaped product is widened, and the strength is temporarily reduced. In this case, it is considered that an internal stress remaining in the shaped product in the shaping step destroys the portion having the reduced strength, thereby causing generation of a crack in the region.

Although the reason why the cracks on the surface of the cured product are not recognized in Patent Document 3 is unclear, the present inventors have conducted an additional experiment on the resin composition for photofabrication described in Patent Document 3, and found that the resin composition for photofabrication for which cracks are observed on the surface of the cured product has low transmittance to activating light. From this, it is considered that in Patent Document 3, the generation of cracks is overlooked.

The present inventors have found that these problems can be solved by controlling a particle diameter of particles constituting the inorganic filler to be blended and setting a transmittance to the specific activating light in the composition before curing to a specific range, and have proposed a novel curable composition for three-dimensional photofabrication (Japanese Patent Application No. 2023-018503). As a preferred embodiment thereof, a curable composition for three-dimensional photofabrication (hereinafter, also referred to as "proposed curable composition") having the following feature points 1 to 4 is proposed.

Feature point 1: the curable composition contains: 100 parts by mass of a polymerizable monomer component (A); 40 parts by mass to 400 parts by mass of an inorganic filler (B) containing a single type or a plurality of types of inorganic particulate matters; 0.01 parts by mass to 5 parts by mass of a photopolymerization initiator (C) having a function of initiating photopolymerization by irradiation with the specific activating light; and 0.01 parts by mass to 2.5 parts by mass of an activating light absorbent (D) having a function of absorbing the specific activating light and having no photopolymerization initiation ability.

Feature point 2: 80% or more of all primary particles constituting the inorganic filler (B) are particles having a particle diameter of 0.05 µm to 5.0 µm in a particle size distribution of the inorganic filler (B) measured by a microscopy method using a scanning microscope.

Feature point 3: a transmittance to the specific activating light measured for a sample having a thickness of 0.5 mm and containing the curable composition for three-dimensional photofabrication is 1% to 50%.

Feature point 4: the polymerizable monomer component (A) and the inorganic filler (B) being such that a value of a light scattering index Sc (%) of a base composition is 10 (%) or less, the base composition being a composition containing only the polymerizable monomer component (A) and the inorganic filler (B) and having a composition ratio of the components same as a composition ratio of the curable composition for three-dimensional photofabrication, the light scattering index Sc (%) being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength λ (nm), including light having a wavelength in a range of λ ± 50 (nm) as a main component, and having a maximum intensity in the range, $Sc = \left\{\left({I}_{70}+{I}_{75}+{I}_{80}\right)/\left({I}_{0}\times 3\right)\right\} \times 100$ wherein I₀, I₇₀, I₇₅, and I₈₀ indicate intensities of the transmitted light in directions of emission angles of 0°, 70°, 75°, and 80°, respectively.

In the proposed curable composition, when the polymerizable monomer component and the inorganic filler are contained at a mass ratio satisfying the conditions shown in the feature point 1, the strength and the surface hardness of the cured product to be a three-dimensional photofabricated product as a production target can be increased. In addition, when the particles constituting the inorganic filler satisfy the conditions shown in the feature point 2 in relation to the particle diameter, it is possible to suppress an increase in viscosity of the composition and a risk of sedimentation of particles during storage. Further, when the conditions shown in the feature points 3 and 4 are satisfied, it is possible to obtain effects of high strength, crack generation suppression, and high accuracy.

In order to further improve the fluidity of the proposed curable composition to improve the operability at the time of use (at the time of performing three-dimensional photofabrication by vat photopolymerization process), the present inventors applied the technique disclosed in Patent Document 4 and conducted studies on blending a part of the inorganic filler (B) as an organic-inorganic composite filler containing particles formed of a composite in which inorganic particulate matters are dispersed in a resin matrix. As a result, it was found that although the fluidity can be improved, the effect of crack generation suppression, which is a characteristic of the proposed curable composition, may be impaired.

Therefore, as a result of further studies to solve the newly confirmed problems, it has been found that the problems can be solved by controlling a refractive index to specific activating light of the polymerizable monomer component, the inorganic filler, and the inorganic particulate matter and resin constituting the organic-inorganic composite filler to be blended, and the present invention has been completed.

That is, the curable composition for photofabrication according to the present disclosure has the following feature points [1] to [5] because the organic-inorganic composite filler is used as an essential component. By having the feature points, the fluidity thereof is further improved while maintaining a characteristic of the proposed curable composition that "it is possible to produce a three-dimensional photofabricated product having a low risk of a decrease in fluidity and a low risk of sedimentation of an inorganic filler and having excellent mechanical strength and good shape accuracy while suppressing generation of cracks on a surface".

Feature point [1] (same as feature point 1): the curable composition contains: 100 parts by mass of a polymerizable monomer component (A); 40 parts by mass to 400 parts by mass of an inorganic filler (B) containing a single type or a plurality of types of inorganic particulate matters; 0.01 parts by mass to 5 parts by mass of a photopolymerization initiator (C) having a function of initiating photopolymerization by irradiation with the specific activating light; and 0.01 parts by mass to 2.5 parts by mass of an activating light absorbent (D) having a function of absorbing the specific activating light and having no photopolymerization initiation ability.

Feature point [2] (same as feature point 2): 80% or more of all primary particles constituting the inorganic filler (B) are particles having a particle diameter of 0.05 µm to 5.0 µm in a particle size distribution of the inorganic filler (B) measured by a microscopy method using a scanning microscope.

Feature point [3]: 10 mass% to 90 mass% of the inorganic filler (B) is blended as an organic-inorganic composite filler (E) containing particles formed of a composite material of the inorganic filler (B) and a resin (R). When, among the inorganic filler (B), an inorganic filler not combined with the resin (R) is defined as a non-composite inorganic filler (B1) and an inorganic filler contained as the organic-inorganic composite filler (E) is defined as a composite inorganic filler (B2), both the non-composite inorganic filler (B1) and the composite inorganic filler (B2) satisfy a condition of the feature point [2]. Compositions and particle size distributions of the non-composite inorganic filler (B1) and the composite inorganic filler (B2) may be the same or different, but since inorganic particulate matters prepared as the inorganic filler (B) (or prepared by mixing a plurality of types of inorganic particulate matters) are generally used separately, the compositions, the particle size distributions, and average primary particle diameters of both are preferably the same or substantially the same.

Feature point [4] (corresponding to feature point 4): the polymerizable monomer component (A), the non-composite inorganic filler (B1), and the organic-inorganic composite filler (E) being such that a value of a light scattering index Sc (%) of a base composition is 10 (%) or less, the base composition being a composition containing only the polymerizable monomer component (A), the non-composite inorganic filler (B1), and the organic-inorganic composite filler (E) and having a composition ratio of the components same as a composition ratio of the curable composition for three-dimensional photofabrication according to the present disclosure, the light scattering index Sc (%) being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength λ (nm), including light having a wavelength in a range of λ ± 50 (nm) as a main component, and having a maximum intensity in the range, $Sc = \left\{\left({I}_{70}+{I}_{75}+{I}_{80}\right)/\left({I}_{0}\times 3\right)\right\} \times 100$ wherein I₀, I₇₀, I₇₅, and I₈₀ indicate intensities of the transmitted light in directions of emission angles of 0°, 70°, 75°, and 80°, respectively.

Feature point [5] (same as feature point 3): a transmittance to the specific activating light measured for a sample having a thickness of 0.5 mm and containing the curable composition for three-dimensional photofabrication is 1% to 50%.

Hereinafter, the feature points [1] to [5] of the curable composition for photofabrication according to the present disclosure will be described together with each component of the curable composition for photofabrication of the present disclosure. In the present description, unless otherwise specified, a notation "x to y" using numerical values x and y means "x or more and y or less". In such a notation, when a unit is added to only the numerical value y, the unit is also applied to the numerical value x. In the present description, the term "(meth)acrylate" means both "acrylate" and "methacrylate". The same applies to terms such as "(meth)acryloyl".

### 2. Regarding Feature Point [1]

The curable composition for photofabrication according to the present disclosure contains: 100 parts by mass of a polymerizable monomer component (A); 40 parts by mass to 400 parts by mass of an inorganic filler (B) containing a single type or a plurality of types of inorganic particulate matters; 0.01 parts by mass to 5 parts by mass of a photopolymerization initiator (C) having a function of initiating photopolymerization by irradiation with the specific activating light; and 0.01 parts by mass to 2.5 parts by mass of an activating light absorbent (D) having a function of absorbing the specific activating light and having no photopolymerization initiation ability.

### 3. Regarding Polymerizable Monomer Component (A)

As the polymerizable monomer component (A), a (meth)acrylate monomer is preferably used from the viewpoint of having a high curing rate and excellent strength of an obtained fabricated product, and from the viewpoint of being able to produce a fabricated product having higher strength, it is preferable that 50 mass% or more, preferably 80 mass% or more, and more preferably 95 mass% or more of the polymerizable monomer component (A) based on a total mass of all radical polymerizable monomers is a bifunctional or higher polyfunctional (meth)acrylate.

Examples of the bifunctional or higher polyfunctional (meth)acrylate preferably used include bisphenol A skeleton-containing (meth)acrylates such as 2,2'-bis{4-[3-(meth)acryloyloxy-2-hydroxypropoxy]phenyl}propane, 2,2'-bis[4-(meth)acryloyloxyphenyl]propane, and 2,2'-bis[4-(meth)acryloyloxypolyethoxyphenyl]propane; ethylene glycol (meth)acrylates such as triethylene glycol dimethacrylate and ethylene glycol dimethacrylate; aliphatic di(meth)acrylates such as 1,3-propanediol di(meth)acrylate and 1,9-nonanediol dimethacrylate; urethane group-containing (meth)acrylates such as 1,6-bis(methacryloyloxy-2-ethoxycarbonyl amino)-2,2,4-trimethylhexane; trifunctional (meth)acrylates such as trimethylol propane trimethacrylate; and isocyanate skeleton-containing (meth)acrylates such as tris(2-methacryloyloxyethyl)isocyanurate. Among them, 2,2'-bis[4-(meth)acryloyloxyphenyl]propane, 2,2'-bis[4-(meth)acryloyloxypolyethoxyphenyl]propane, triethylene glycol dimethacrylate, tris(2-methacryloyloxyethyl)isocyanurate, and the like are preferable from the viewpoint of low viscosity and high strength.

Examples of monofunctional (meth)acrylates suitable for use in combination with bifunctional or higher polyfunctional (meth)acrylates include hydroxyethylmethacrylate, methyl(meth)acrylate, ethyl(meth)acrylate, isopropyl(meth)acrylate, hydroxyethyl(meth)acrylate, tetrahydrofurfuryl(meth)acrylate, and glycidyl(meth)acrylate.

As the polymerizable monomer component (A), only one type of the (meth)acrylates may be used, or a plurality of types thereof may be used in combination.

### 4. Regarding Inorganic Filler (B)

In order to increase the mechanical strength such as rigidity of the obtained fabricated product, the curable composition for photofabrication according to the present disclosure contains 40 parts by mass to 400 parts by mass of the inorganic filler (B) with respect to 100 parts by mass of the polymerizable monomer component (A). When a content of the inorganic filler (B) is too large, the viscosity of the composition becomes too high. On the other hand, when the content of the inorganic filler (B) is too small, the mechanical strength becomes insufficient. Therefore, the content of the inorganic filler (B) is preferably 50 parts by mass to 350 parts by mass, and more preferably 60 parts by mass to 300 parts by mass with respect to 100 parts by mass of the polymerizable monomer component (A).

As shown in the feature point [2], in the inorganic filler (B), 80% or more (preferably 90% or more, and more preferably 95% or more) of all primary particles constituting the inorganic filler (B) needs to be particles having a particle diameter of 0.05 µm to 5.0 µm in a particle size distribution measured by a microscopy method using a scanning microscope.

Here, the fact that 80% or more of all the primary particles constituting the inorganic filler (B) are particles having a particle diameter of 0.05 µm to 5.0 µm can be found by the particle size distribution of the inorganic filler (B) measured by the microscopy method using a scanning microscope. That is, the particle size distribution can be determined by photographing, by a scanning electron microscope, the inorganic filler (B), measuring the number n of all primary particles (50 or more) observed in a unit field of view of the photograph, and measuring a primary particle diameter (maximum diameter) Xᵢ (nm) of each particle for all particles. Here, i in Xᵢ is a natural number of 1 to n, and represents the number of each measured primary particle.

Based on measured values obtained in this manner, the average primary particle diameter X (nm) can be calculated and confirmed by an equation X = ΣXᵢ/n using a total sum ΣXᵢ of the measured particle diameters (of primary particles where i = 1 to n) of all the primary particles.

The average primary particle diameter of the inorganic filler (B) is preferably 0.07 µm to 3 µm, and more preferably 0.08 µm to 1 µm.

When the inorganic filler (B) having such a particle size distribution is contained at the above-described mass ratio, the strength and the surface hardness of the cured product to be a three-dimensional photofabricated product as a production target may be increased. In addition, when the particles constituting the inorganic filler (B) satisfy the above conditions in relation to the particle diameter, it is possible to suppress an increase in viscosity of the composition and a risk of sedimentation of particles during storage. From the viewpoint of further suppressing an increase in the viscosity, a lower limit value of the particle diameter of the particles occupying 80% or more of the inorganic filler (B) is preferably 0.08 µm, and more preferably 0.1 µm. From the viewpoint of further suppressing sedimentation, an upper limit value of the particle diameter of the particles occupying 80% or more of the inorganic filler (B) is preferably 2.0 µm, and more preferably 1.0 µm.

The inorganic filler (B) may be contained as aggregated particles in which primary particles are aggregated. The particle size distribution of the aggregated particles is not particularly limited as long as the particle size distribution of the primary particles satisfies the above conditions, but in order to suppress the sedimentation of the inorganic filler (B), the amount of the aggregated particles of the inorganic filler (B) contained in the curable composition for photofabrication according to the present disclosure is preferably small. An average particle diameter of the inorganic filler (B) containing the aggregated particles of the primary particles measured by a laser diffraction/scattering method is generally 0.05 µm to 100 µm, preferably 0.05 µm to 50 µm, and more preferably 0.05 µm to 30 µm.

As shown in the feature point [3], the inorganic filler (B) is divided into the non-composite inorganic filler (B1) that is blended as it is without being combined with the resin (R) and a composite inorganic filler (B2) that is blended as the organic-inorganic composite filler (E), and thus a blending amount of the inorganic filler (B) means a total blending amount of the non-composite inorganic filler (B1) and the composite inorganic filler (B2) with respect to 100 parts by mass of the polymerizable monomer component (A).

As shown in the feature point [3], 10 mass% to 90 mass% of the inorganic filler (B) is the composite inorganic filler (B2), and the remainder is the non-composite inorganic filler (B1). When a ratio of the composite inorganic filler (B2) in the inorganic filler (B) is too large, the fluidity is significantly reduced. On the other hand, when the ratio of the composite inorganic filler (B2) in the inorganic filler (B) is too small, it is difficult to obtain an effect of improving the fluidity by adding the organic-inorganic composite filler (E). Therefore, the ratio of the composite inorganic filler (B2) in the inorganic filler (B) is preferably 20 mass% to 80 mass%, and more preferably 30 mass% to 70 mass%.

80% or more (preferably 90% or more, and more preferably 95% or more) of all the primary particles constituting the non-composite inorganic filler (B1) and the composite inorganic filler (B2) are particles having a particle diameter of 0.05 µm to 5.0 µm. Compositions and particle size distributions of the non-composite inorganic filler (B1) and the composite inorganic filler (B2) may be the same or different, but since inorganic particulate matters prepared as the inorganic filler (B) (or prepared by mixing a plurality of types of inorganic particulate matters) are generally used separately, the compositions, the particle size distributions, and average primary particle diameters of both are preferably the same or substantially the same. The term "substantially the same" basically means that a difference is within a measurement error range.

As the inorganic filler (B), under the assumption that the condition shown in the feature point [5] can be satisfied and the condition shown in the feature point [4] can be satisfied, for example, the inorganic particulate matters to be used as the inorganic filler in a tooth restoration material can be used without particular limitation. From the viewpoint of easily satisfying the assumption, it is preferable to use, as the inorganic particulate matters, particles formed of amorphous silica; silica-based composite oxides such as silica-zirconia, silica-titania, silica-titania-barium oxide, and silica-titania-zirconia; and glasses such as borosilicate glass, aluminosilicate glass, and fluoroaluminosilicate glass. In addition, from the viewpoint of having X-ray contrast properties, it is more preferable to use particles formed of silica-based composite oxides such as silica-zirconia, silica-titania, silica-titania-barium oxide, and silica-titania-zirconia. Further, from the viewpoint of wear resistance of the cured product, it is more preferable to use particles formed of silica-zirconia particles.

The inorganic particulate matters are preferably treated with any surface treatment agent represented by a silane coupling agent in order to improve compatibility with the polymerizable monomer component (A) and improve the mechanical strength and water resistance. The surface treatment may be performed by a known method. Examples of the silane coupling agent include methyltrimethoxysilane, methyltriethoxysilane, methyltrichlorosilane, dimethyldichlorosilane, trimethylchlorosilane, vinyltrichlorosilane, vinyltriethoxysilane, vinyltris(β-methoxyethoxy)silane, γ-methacryloyloxypropyltrimethoxysilane, methacryloxyoctyl-8-trimethoxysilane, γ-chloropropyltrimethoxysilane, γ-glycidoxypropylmethoxysilane, and hexamethyldisilazane.

### 5. Regarding Photopolymerization Initiator (C)

The photopolymerization initiator (C) needs to have a function of radically polymerizing a polymerizable monomer component by generating a radical with specific activating light including light of a specific wavelength λ (nm) in an ultraviolet light range or a visible light range emitted from a light source mounted on a photofabrication device. In other words, the photopolymerization initiator (C) needs to absorb the light having a specific wavelength λ (nm) to generate radicals. The specific wavelength λ may be appropriately determined according to a wavelength of the activating light used in the photofabrication device as long as it is a wavelength in the ultraviolet light range or the visible light range. Examples of a general-purpose photofabrication device include an SLA photofabrication device that emits semiconductor laser light as the activating light, a DLP photofabrication device that emits projector light, and an LCD photofabrication device that emits liquid crystal panel light, and a light source having an activating light wavelength of 405 nm or 385 nm is often used. In the present disclosure, the specific wavelength λ is preferably 405 nm or 385 nm, and the photofabrication device is preferably SLA, DLP, or LCD.

A blending amount of the photopolymerization initiator (C) may be 0.05 parts by mass to 5.0 parts by mass with respect to 100 parts by mass of the polymerizable monomer component (A). When the blending amount of the photopolymerization initiator (C) is too large, burrs or the like are generated in the obtained fabricated product, and the accuracy is deteriorated. On the other hand, when the blending amount of the photopolymerization initiator (C) is too small, fabrication cannot be performed in the shaping step. Therefore, the blending amount of the photopolymerization initiator (C) is preferably 0.3 parts by mass to 4.0 parts by mass, and more preferably 0.5 parts by mass to 3.0 parts by mass with respect to 100 parts by mass of the polymerizable monomer component (A).

As the photopolymerization initiator (C), a known photopolymerization initiator satisfying the above conditions may be appropriately selected and used. The photopolymerization initiator to be selected is not particularly limited, and examples thereof include self-cleaving photopolymerization initiators, bimolecularly hydrogen-abstracting photopolymerization initiators, photoacid generators, and combinations thereof. The photopolymerization initiators may be used in combination with a photosensitizing dye, an electron-donating compound, or the like.

Examples of self-cleaving photopolymerization initiators that can be suitably used include acylphosphine oxide compounds such as diphenyl(2,4,6-trimethylbenzoyl)-phosphine oxide and phenyl bis(2,4,6-trimethylbenzoyl)-phosphine oxide; benzoketal compounds, benzyne compounds, α-aminoacetophenone compounds, α-hydroxyacetophenone compounds, titanocene compounds, and acyloxime compounds. Examples of the photoacid generators include iodonium salt compounds such as p-isopropylphenyl-p-methylphenyliodonium tetrakispentafluorophenyl borate salt; sulfonium salt compounds such as dimethylphenacylsulfonium hexafluoroantimonate salt; and halomethyl group-substituted triazine compounds such as 2,4,6-tris(trichloromethyl)-s-triazine. Examples of the photosensitizing dye include ketone compounds, coumarin dyes, cyanine dyes, merocyanine dyes, thiazine dyes, azine dyes, acridine dyes, xanthene dyes, squarylium dyes, pyrylium salt dyes, fused polycyclic aromatic compounds (anthracene, perylene, etc.), and thioxanthone compounds. Examples of the electron-donating compound include 4-dimethylaminobenzoic acid ester, 4-dimethylaminotoluene, p-dimethoxybenzene, 1,2,4-trimethoxybenzene, and thiophene compounds.

### 6. Regarding Activating Light Absorbent (D)

When a shaped product such as a dental prosthesis is produced with high accuracy by using the curable composition for photofabrication according to the present disclosure, in order to prevent the activating light emitted from the photofabrication device from being excessively transmitted and the fabrication accuracy of the shaped product from being lowered, 0.01 parts by mass to 2.5 parts by mass of the activating light absorbent (D) that has a function of absorbing the activating light emitted from the photofabrication device and does not function as a photopolymerization initiator is blended with respect to 100 parts by mass of the polymerizable monomer component.

When a blending amount of the activating light absorbent (D) is too large, the light generated from the light source of the photofabrication device in the shaping step does not pass through the composition, and the condition shown in the feature point [5] cannot be satisfied. On the other hand, when the blending amount of the activating light absorbent (D) is too small, the accuracy of the obtained fabricated product is lowered. Therefore, the blending amount of the activating light absorbent (D) is preferably 0.04 parts by mass to 2.5 parts by mass, more preferably 0.08 parts by mass to 2.0 parts by mass, and further preferably 0.25 parts by mass to 1.0 part by mass with respect to 100 parts by mass of the polymerizable monomer component.

The activating light absorbent (D) is not particularly limited as long as it is a compound that absorbs light emitted from the light source mounted on the photofabrication device, and examples thereof include triazole compounds such as 2-(hydroxy-5-methylphenyl)-2H-benzotriazole and 2-(3-tert-butyl-2-hydroxy-5-methylphenyl)-5-chloro-2H-benzotriazole; and benzophenone compounds such as 2,4-dihydroxybenzophenone and 2-hydroxy-4-methoxybenzophenone.

### 7. Regarding Organic-inorganic Composite Particulate Matter (E)

As shown in the feature point [3], the composite inorganic filler (B2) occupying 10 mass% to 90 mass%, preferably 20 mass% to 80 mass%, and more preferably 30 mass% to 70 mass% of the inorganic filler (B) is blended as the organic-inorganic composite filler (E).

Here, the organic-inorganic composite filler means a particulate matter consisting of particles formed of a composite material of the composite inorganic filler (B2) and the resin (R). Examples of a form of the composite material include a first form in which the composite inorganic filler (B2) is uniformly dispersed in the matrix of the resin (R); and a second form in which a surface of each of inorganic primary particles constituting the composite inorganic filler (B2) is covered with a resin and the inorganic primary particles are bonded to each other by the resin to form (microporous) aggregated particles. Regarding content ratios of the composite inorganic filler (B2) and the resin (R) in the composite material, the ratio of the composite inorganic filler (B2) in the composite material is preferably 50 mass% to 99 mass%, and more preferably 70 mass% to 95 mass%. In addition, an average particle diameter of the organic-inorganic composite filler (E), specifically, an average particle diameter measured by a laser diffraction/scattering method is preferably 0.5 µm to 100 µm, more preferably 1 µm to 50 µm, and further preferably 2 µm to 30 µm.

It is well known that the organic-inorganic composite filler of the first form or the second form can be produced by using a polymerizable monomer component serving as a raw material of an inorganic particulate matter and the resin (R), and the organic-inorganic composite filler (E) can also be produced by using the polymerizable monomer component serving as the raw material of the composite inorganic filler (B2) and the resin (R). As the polymerizable monomer component, those exemplified as the polymerizable monomer component (A) are suitably used, but the polymerizable monomer component is not necessarily the same as the polymerizable monomer component (A).

### 8. Regarding Other Components

### (Polymerization Inhibitor)

The curable composition for photofabrication according to the present disclosure is preferably blended with a polymerization inhibitor in an amount of 0.01 parts by mass to 5.0 parts by mass with respect to 100 parts by mass of the polymerizable monomer component (A) for the purpose of improving storage stability. When a blending amount of the polymerization inhibitor is too large, the composition is insufficiently cured in the shaping step. On the other hand, when the blending amount of the polymerization inhibitor is too small, the storage stability and the fabrication accuracy are reduced. Therefore, the blending amount of the polymerization inhibitor is preferably 0.01 parts by mass to 5.0 parts by mass, more preferably 0.03 parts by mass to 4.0 parts by mass, and further preferably 0.05 parts by mass to 2.5 parts by mass with respect to 100 parts by mass of the polymerizable monomer component (A).

As the polymerization inhibitor, a compound which reacts with the radical generated in the curable composition for photofabrication to deactivate the radical can be used, and examples thereof include di-tert-butyl-p-cresol and 4-methoxyphenol.

### (Chain Transfer Agent)

The curable composition for photofabrication according to the present disclosure may be blended with a chain transfer agent in a range of 0.00001 parts by mass to 1.0 part by mass with respect to 100 parts by mass of the polymerizable monomer component (A) for the purpose of reducing shrinkage stress during photofabrication and improving fabrication accuracy. When a blending amount of the chain transfer agent is too large, a polymerization reaction of the curable composition for photofabrication is suppressed more than necessary, and when the blending amount of the chain transfer agent is too small, an effect of adding the chain transfer agent cannot be exerted.

Examples of the chain transfer agent include thiol compounds such as butanethiol, thiophenol, mercaptoethanol, octylthiol, lauryl mercaptan; α-alkylstyrene compounds such as 2,4-diphenyl-4-methyl-1-pentene (α-methylstyrene dimer), 2-phenyl-1-propene (α-methylstyrene); and halogenated hydrocarbon substituted with at least one halogen atom such as carbon tetrachloride and ethylene bromide. Among them, an α-alkylstyrene compound, particularly an α-methylstyrene dimer, is preferably used from the viewpoint that the effect of suppressing the generation of cracks is high.

### (Thermal Polymerization Initiator)

The curable composition for photofabrication according to the present disclosure may be blended with a thermal polymerization initiator as a polymerization initiator for secondary curing. In this case, a thermal polymerization initiator having a 10-hour half-life temperature of 50°C to 130°C is preferably used from the viewpoint of effectively remaining in the stack without functioning during primary curing. Examples of the thermal polymerization initiator that can be suitably used include organic peroxides such as tert-butyl peroxylaurate and benzoyl peroxide; and azo compounds such as azobutylonitrile and azobis(dimethylvaleronitrile).

A blending amount of the thermal polymerization initiator is generally 0.001 parts by mass to 1.0 part by mass, preferably 0.005 parts by mass to 0.3 parts by mass, and more preferably 0.01 parts by mass to 0.1 parts by mass with respect to 100 parts by mass of the polymerizable monomer component (A).

### (Coloring Substance)

The curable composition for photofabrication according to the present disclosure may be blended with a coloring substance in a range in which the conditions shown in the feature points [4] and [5] are satisfied. For example, when the curable composition for photofabrication according to the present disclosure is used to produce a dental restoration such as an inlay, an onlay, a crown, or a denture, it is preferable to blend a coloring substance in order to reproduce a color of a crown or oral mucosa. The coloring substance may be a pigment or a dye. Examples of the pigment include inorganic pigments such as titanium oxide, zinc oxide, zirconium oxide, zinc sulfide, aluminum silicate, calcium silicate, carbon black, iron oxide, copper chromite black, chromium oxide green, chromium green, violet, chromium yellow, lead chromate, lead molybdate, cadmium titanate, nickel titanium yellow, ultramarine blue, cobalt blue, bismuth vanadate, cadmium yellow, and cadmium yellow; and organic pigments such as a monoazo pigment, a diazo pigment, a diazo fused pigment, a perylene pigment, and an anthraquinone pigment.

### 9. Conditions to be satisfied by Components (A) to (E): Regarding Feature Points [4] and [5]

In the curable composition for photofabrication according to the present disclosure, it is important to satisfy the conditions shown in the feature points [4] and [5] in order to reduce the risk of generation of cracks on the surface of the cured product. That is, by satisfying the condition shown in the feature point [4], the condition shown in the feature point [5] is easily satisfied, and by satisfying the condition shown in the feature point [5], the risk of generation of cracks on the surface of the cured product can be reduced. When the condition shown in the feature point [5] is not satisfied, that is, when the transmittance is less than 1%, a sufficient curing depth is not obtained, and thus a large number of interlayer low-crosslinking-density regions are formed, and the generation of cracks becomes remarkable. On the other hand, in a composition containing the inorganic filler (B) and the activating light absorbent (D), it is very difficult to obtain a system in which the transmittance exceeds 50% while maintaining high fabrication accuracy.

In the curable composition for photofabrication according to the present disclosure, the transmittance to the specific activating light is set within the above range, so that an influence of scattering is reduced, and the risk of generation of cracks on the surface of the cured product is reduced, and in order to more reliably suppress generation of cracks and produce a shaped object with high accuracy, a combination in which the light scattering index Sc is within a specific range is used as a combination of the polymerizable monomer component (A), the non-composite inorganic filler (B1), and the organic-inorganic composite filler (E). According to the curable composition for photofabrication of the present disclosure, light scattering due to the inorganic filler in a direction deviated from the optical axis is suppressed and the transmittance to the specific activating light (in state before curing) is set within a specific range, so that the generation of cracks can be suppressed, and in some cases, the fabrication accuracy can be further improved.

The conditions shown in the feature points [4] and [5] define conditions to be satisfied by the polymerizable monomer component (A), the inorganic filler (B), the photopolymerization initiator (C), the activating light absorbent (D), and the organic-inorganic composite filler (E), and the transmittance defined in the feature point [5] corresponds to the "transmittance to specific activating light in the curable composition for photofabrication according to the present disclosure in a state before photocuring", and is an index of the curing depth when the curable composition for photofabrication according to the present disclosure is irradiated with the specific activating light. In addition, Sc defined in the feature point [4] is an index of "a scattering state of light when the curable composition for photofabrication according to the present disclosure is irradiated with the specific activating light", specifically, the state of the side scattered light, and further, the spread of the interlayer low-crosslinking-density region formed thereby. The curing depth and the state of the side scattered light are important for development of the effect of the curable composition for photofabrication according to the present disclosure, and are determined by the combination of the components to be used. However, since not only physical properties of each component affecting the transmittance and Sc are various, but also a relation with physical properties of other components is affected, it is substantially impossible to directly define a combination of specific substances satisfying these conditions. Therefore, the curable composition for photofabrication according to the present disclosure is defined to be simultaneously satisfying the conditions shown in the feature points [4] and [5]. As described above, it is substantially impossible for the curable composition for photofabrication according to the present disclosure to be defined only by components constituting the composition and a blending amount thereof, and the curable composition for photofabrication has to be defined by parameters shown in the feature points [4] and [5] or defined as the curable composition for photofabrication obtained by the production method according to the present disclosure.

As described above, since it is important to satisfy the conditions shown in the feature points [4] and [5] from the viewpoint of realizing the effect of the present invention, particularly, a crack suppression effect and moderate transparency, the feature points will be described in detail below.

### 9-1. Regarding Feature Point [4]

In general, it is known that when light is incident on fine particles having a particle diameter suitable for the resin composition for photofabrication, that is, fine particles having a particle diameter of 0.05 µm to 5.0 µm, phenomena such as light shielding, diffraction, Mie scattering, and Rayleigh scattering occur. Among these, when Mie scattering and/or Rayleigh scattering occurs, the scattered light spreads not only forward but also laterally and rearward. Then, it is considered that the scattered light spread laterally (side scattered light) reduces the transmittance to the specific activating light set in the feature point [5], which causes the generation of cracks, and further reduces the fabrication accuracy of the shaped product.

The spread and intensity of the side scattered light, that is, an intensity distribution of the scattered light in a scattering direction (angle) is affected not only by the particle diameter of the particle, but also by the refractive index of the particle and the polymerizable monomer component present around the particle (serving as dispersion medium). However, in a dispersion system in which particles are dispersed in a polymerizable monomer component as particulate matters having the particle size distribution, particularly in a dispersion system constituted by a plurality of types of particles having different refractive indexes, it is substantially impossible to grasp a scattering behavior of each particle, and it is necessary to grasp a scattering behavior of the entire system. In addition, when the curable composition for photofabrication according to the present disclosure is used for production of a dental prosthetic material, as an average "intensity of activating light scattered in a lateral direction" of the entire system is larger, the fabrication accuracy is reduced, the transmittance to the specific activating light is reduced, and generation of cracks due to an increase of the interlayer low-crosslinking-density region is concerned. For this reason, in the present disclosure, the combination of the polymerizable monomer, the non-composite inorganic filler, and the organic-inorganic composite filler used in the curable composition for photofabrication according to the present disclosure is indirectly defined by using the light scattering index Sc (%) defined by the above formula in the base composition (which is uniform, consists of polymerizable monomer, non-composite inorganic filler, and organic-inorganic composite filler at predetermined mass ratio) serving as a base of the curable composition for photofabrication according to the present disclosure.

The measurement of the refracted light transmitted through the sample using a variable angle spectrophotometer (goniophotometer) is used, for example, for evaluation of optical properties of a material requiring light diffusibility, such as a cover of a lighting fixture or a projector screen, and is also used for determination of an index for evaluating an optical texture of a tooth substance filling restoration material, specifically, a diffusivity D, as described in Patent Document 5 in the dental field.

In the present disclosure, measurement using a variable angle spectrophotometer for determining the light scattering index Sc can be performed as follows. First, a measurement sample having a thickness of 0.5 mm is prepared in the same manner as in the measurement of transmittance, by using a (measurement) base composition prepared by sampling or separately preparing a part of a (raw material) base composition prepared in the preparation process of the curable composition for photofabrication according to the present disclosure. Next, the sample is set in a three-dimensional variable angle spectrophotometer (for example, GP-200 manufactured by Murakami Color Research Laboratory Co., Ltd.) and is vertically irradiated with "measurement light including light having a specific wavelength λ (nm), including light having a wavelength in a range of λ ± 50 (nm) as a main component, and having a maximum intensity in the range" to measure the intensity of transmitted light in each emission angle direction. When light emitted from a light source (capable of emitting light including light having wavelength λ) attached to the three-dimensional variable angle spectrophotometer does not satisfy the condition related to the measurement light, the light may be set to measurement light satisfying the above-described condition using an interference filter (for example, GP-200 manufactured by Murakami Color Research Laboratory Co., Ltd.). The fact that the light having a wavelength in the range of λ ± 50 (nm) is the main component means that an integrated value of the intensity of the light having the wavelength of λ ± 50 (nm) in a spectrum showing the wavelength distribution (relative spectral distribution) of the measurement light is 90% or more of an integrated value of the intensity of the entire measurement light.

When the light scattering index Sc of the base composition is more than 10%, the formation of the interlayer low-crosslinking-density region, which is a cause of generation of cracks due to the side scattered light, cannot be avoided. From the viewpoint of improving the fabrication accuracy and the effect of crack generation suppression, the light scattering index Sc of the base composition is preferably 5.0% or less, and more preferably 3.0% or less. The light scattering index Sc is preferably as low as possible, and a lower limit value thereof is 0.0%.

The light scattering index Sc of the base composition is affected by the blending ratio of the polymerizable monomer component (A), the non-composite inorganic filler (B1), and the organic-inorganic composite filler (E), and when the ratio is constant, the light scattering index Sc may be controlled to a certain extent by adjusting the particle size distribution of the non-composite inorganic filler (B1) and the composite inorganic filler (B2) in the organic-inorganic composite filler (E), the refractive index (or type) of the particles constituting the inorganic particulate matter, the refractive index of the entire polymerizable monomer component, and the refractive index of the resin (R) in the organic-inorganic composite filler (E).

For example, regarding the particle diameter, a particle diameter parameter called "particle size parameter α" which is an index of scattering or scattering intensity of light caused by particles is known. When a wavelength (nm) of an incident light is defined as λ₀, the circumference ratio is defined as π, and a particle diameter of inorganic particles is defined as x (nm), the particle diameter parameter α is defined by the formula α = (π × x)/λ₀. In a case where the particles are transparent to the incident light, it is considered that diffraction occurs when the particle size parameter α exceeds 10, and Mie scattering or Rayleigh scattering occurs when the particle size parameter α is 10 or less. When laser light having a wavelength of 405 nm, which is the most common activating light in the vat photopolymerization process, is used, the particle diameter x at which α = 10 is obtained is 1,290 nm = 1.29 µm based on a relation of x = α·λ₀/π, and when laser light having a wavelength of 385 nm is used, the particle diameter x at which α = 10 is obtained is 1,226 nm = 1.23 µm. Since many of dental inorganic fillers have a particle diameter smaller than the value in many cases, it can be said that the scattering occurs and cracks are likely to be generated.

According to the study by the present inventors, even when Mie scattering or Rayleigh scattering occurs, the light scattering index Sc tends to decrease as a ratio of particles having a particle diameter in which the particle diameter parameter α is in a range of 0.7 to 4, that is, particles satisfying a relation of x = 0.7 × (λ/π) to 4 × (λ/π) (when wavelength of activating light is 405 nm, x = 90 nm to 514 nm, and when wavelength of activating light is 385 nm, x = 86 nm to 490 nm) increases in the particle size distribution measured by the microscopy method using a scanning microscope. For the reason that it is easy to set the light scattering index Sc to 10% or less, the inorganic filler (B) to be used, that is, the non-composite inorganic filler (B1) and the composite inorganic filler (B2) preferably satisfy the following condition 1.

Condition 1: when a particle diameter of each primary particle constituting the inorganic filler (B) is defined as x (nm) and the circumference ratio is defined as π, a total number of particles having a particle diameter x (nm) within a range of 0.7λ/π to 4λ/π (nm) is 40% or more, preferably 60% or more, and more preferably 80% or more of the number of all primary particles constituting the inorganic filler (B) in the particle size distribution of the inorganic filler (B) measured by a microscopy method using a scanning microscope.

For the reason that the condition in the feature point [4] is more easily satisfied, in the particle size distribution measured by the microscopy method using a scanning microscope, a ratio of particles having a particle diameter (129 nm to 386 nm when wavelength of activating light is 405 nm) in which the particle diameter parameter α in the inorganic filler (B) is in a range of 1.0 to 3.0 is preferably 50% or more, and more preferably 60% or more, and a ratio of particles having a particle diameter (231 nm to 360 nm when wavelength of activating light is 405 nm) in which the particle diameter parameter α is in a range of 1.8 to 2.8 is further preferably 45% or more, and particularly preferably 50% or more.

Since a generally used photofabrication device (3D printer) uses a light source having an activating light wavelength (peak wavelength) of 380 nm to 420 nm, in order to simultaneously satisfy the conditions shown in the feature points [4] and [5], the total number of particles having a particle diameter in a range of 85 nm to 535 nm in the particle size distribution measured by a microscopy method using a scanning microscope is preferably 40% or more, more preferably 60% or more, and further preferably 80% or more of the number of all primary particles constituting the inorganic filler (B). The range of the particle diameter is more preferably 121 nm to 400 nm, and further preferably 218 nm to 375 nm. That is, in the particle size distribution measured by the microscopy method using a scanning microscope, the total number of particles having a particle diameter in the range of 218 nm to 375 nm is most preferably 80% or more of the number of all primary particles constituting the inorganic filler (B).

The light scattering index Sc tends to decrease as a difference between the refractive index of the particles and the refractive index of the polymerizable monomer component decreases. In consideration of the refractive index of the inorganic filler and the refractive index of the polymerizable monomer component for dental use, a combination satisfying the following conditions 2 to 4 is preferably adopted as a combination of the inorganic filler (B) and the polymerizable monomer component (A).

Condition 2: in the inorganic particulate matters constituting the inorganic filler (B), when an inorganic particulate matter consisting of an aggregate of a single type of inorganic particles having a refractive index n_{(D)F} with respect to a (sodium) line D at 25°C in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), each of the non-composite inorganic filler (B1) and the composite inorganic filler (B2)
(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of each of the non-composite inorganic filler (B1) and the composite inorganic filler (B2), or
(3) contains 90 mass% or more and less than 100 mass% of a single type or a plurality of types of the specific inorganic particulate matters (b1) and more than 0 mass% and 10 mass% or less of a single type or a plurality of types of the non-specific inorganic particulate matters (b2), at least one of the single type or the plurality of types of specific inorganic particulate matters (b1) occupying 10 mass% or more of a total mass of each of the non-composite inorganic filler (B1) and the composite inorganic filler (B2).

Condition 3: the refractive index n_{(D)M} of the polymerizable monomer component (A) with respect to the line D at 25°C is within a range of 1.490 to 1.550.

Condition 4: when a refractive index at which a difference with the refractive index n_{(D)M} is largest among the refractive indexes n_{(D)F} with respect to the line D at 25°C of at least one of the specific inorganic particulate matters (b1) occupying 10 mass% or more of the total mass of each of the non-composite inorganic filler (B1) and the composite inorganic filler (B2) is defined as n_{(D)Fh}, an absolute value |n_{(D)Fh} - n_{(D)M}| of the difference between n_{(D)Fh} and n_{(D)M} is 0.035 or less.

When |n_{(D)Fh} - n_{(D)M}| increases, the refraction of the activating light increases at an interface between the inorganic filler and the polymerizable monomer component, the intensity of the side scattered light increases, and the light scattering index Sc tends to increase. When the ratio of particles having a particle diameter considerably smaller than the wavelength of the activating light is high, Rayleigh scattering is likely to occur, and the light scattering index Sc tends to increase. Conversely, when the ratio of particles having a particle diameter considerably larger than the wavelength of the activating light is high, the frequency of collision of the activating light with the inorganic filler is significantly increased, and the light scattering index Sc tends to increase.

The refractive index n_{(D)F} with respect to the line D at 25°C of the inorganic filler (B) {non-composite inorganic filler (B1) and composite inorganic filler (B2)} formed of a single material and the refractive index n_{(D)M} of the polymerizable monomer component with respect to the line D at 25°C can be measured as follows.

That is, the refractive index n_{(D)M} of the polymerizable monomer component can be measured using an Abbe refractometer (for example, digital Abbe refractometer DR-A1-PLUS manufactured by Atago Co., Ltd.) by placing the prepared monomer composition on a prism, then viewing a sample from an eyepiece, and reading a value of a display section when an intersection of a boundary line and a cross line is matched (value becoming refractive index). In addition, the refractive index n_{(D)F} of the inorganic particulate matters constituting the inorganic filler is determined by mixing toluene or ethanol and bromonaphthalene to prepare solutions having refractive indexes different from each other by 0.001, then mixing inorganic particulate matters into the respective solutions having different refractive indexes, shaking the mixture, and setting the refractive index of the solution observed to be most transparent as the refractive index of the inorganic particulate matter.

### 9-2. Regarding Feature Point [5]

In the curable composition for photofabrication according to the present disclosure, a transmittance to the specific activating light measured for a sample having a thickness of 0.5 mm needs to be 1% to 50%. The transmittance is an index of the curing depth, and when the transmittance is lower than 1%, which is a lower limit value, a sufficient curing depth is not obtained, photofabrication is difficult, and cracks are likely to be generated on the fabricated product even when photofabrication is possible. In addition, in a system containing 40 parts by mass or more of an inorganic filler (B) with respect to 100 parts by mass of a polymerizable monomer component (A) and containing a photopolymerization initiator in an amount capable of being cured by photofabrication, it is difficult to obtain a transmittance greatly exceeding 50%, which is an upper limit value. In addition, even in a range of transmittance that may be implemented, since the specific activating light is excessively irradiated, a curing reaction excessively occurs, and a shaped product having a shape completely different from CAD data is provided. From the viewpoint of crack generation suppression and the shape of the shaped product, the transmittance is preferably 2% to 30%, and more preferably 5% to 20%.

Since the curable composition for photofabrication according to the present disclosure contains a photopolymerization initiator, there is a concern about the progress of curing by irradiation with light during measurement. In this regard, it is possible to measure the transmittance to the specific activating light before the progress of curing by using a transmission measurement method using a color difference meter described later.

The transmittance of the curable composition for photofabrication according to the present disclosure to the activating light can be measured as follows. First, a measurement sample having a thickness of 0.5 mm is prepared by a method in which the resin composition for photofabrication according to the present disclosure is filled in a resin mold (25 mm × 25 mm × 0.5 mm in thickness) or the like, then upper and lower surfaces are pressed with a slide glass, and the slide glass is removed after the thickness becomes 0.5 mm. Next, the measurement sample is set in a color difference meter (for example, spectrophotometer SE7700 manufactured by Nippon Denshoku Industries Co., Ltd.), and the transmittance to specific activating light (for example, light having wavelength of 405 nm or light having wavelength of 385 nm) is measured by transmission measurement using measurement light from a halogen lamp (measurement wavelength of 380 nm to 780 nm).

The above transmittance tends to increase as light transmittances of the polymerizable monomer component (A), the inorganic filler (B), and the resin (R) itself constituting the organic-inorganic composite filler (E) increase. In addition, the above transmittance tends to increase as the difference between the refractive index with respect to the specific activating light of the inorganic filler (B) and the refractive index with respect to the specific activating light of the polymerizable monomer component (A), the difference between the refractive index with respect to the specific activating light of the resin (R) and the refractive index with respect to the specific activating light of the polymerizable monomer component (A), and the difference between the refractive index with respect to the specific activating light of the inorganic filler (B) and the refractive index with respect to the specific activating light of the resin (R) decrease. In addition, when the polymerizable monomer component (A), the inorganic filler (B), and the resin (R) have different light transmittances, the above transmittance is affected by a blending ratio thereof. In general, since the above transmittance of the polymerizable monomer component and the resin is considerably higher than 5%, when a blending amount of the inorganic filler is within a defined range, it is possible to sufficiently set the above transmittance not only to 1% or more but also to 3% or more, or 5% or more by using an inorganic filler having transparency and reducing the differences in refractive index described above.

The polymerizable monomer component (A), the inorganic filler (B), and the resin (R) preferably satisfy the following condition 5 in order to easily satisfy the condition of the feature point [5].

Condition 5: the refractive index n_{(AL)F} of at least one of the inorganic particulate matters (b1) occupying 10 mass% or more of the total mass of each of the non-composite inorganic filler (B1) and the composite inorganic filler (B2) with respect to the specific activating light at 25°C, the refractive index n_{(AL)M} of the polymerizable monomer component (A) with respect to the specific activating light at 25°C, and the refractive index n_{(AL)R} of the resin (R) with respect to the specific activating light at 25°C satisfy all relations shown in the following formulae (i) to (iii), $- 0.015 < \left({n}_{\left(AL\right) F}-{n}_{\left(AL\right) M}\right) < 0.025$ (i), $- 0.015 < \left({n}_{\left(AL\right) R}-{n}_{\left(AL\right) M}\right) < 0.030$ (ii), and $- 0.020 < \left({n}_{\left(AL\right) F}-{n}_{\left(AL\right) R}\right) < 0.020$ (iii).

In condition 5, it is preferable that all conditions shown in the following (ia) to (iiia) are satisfied, $- 0.012 < \left({n}_{\left(AL\right) F}-{n}_{\left(AL\right) M}\right) < 0.022$ (ia), $- 0.010 < \left({n}_{\left(AL\right) R}-{n}_{\left(AL\right) M}\right) < 0.022$ (iia), and $- 0.020 < \left({n}_{\left(AL\right) F}-{n}_{\left(AL\right) R}\right) < 0.017$ (iiia),

In addition, it is more preferable that all conditions shown in the following (ib) to (iiib) are satisfied, $- 0.005 < \left({n}_{\left(AL\right) F}-{n}_{\left(AL\right) M}\right) < 0.015$ (ib), $- 0.005 < \left({n}_{\left(AL\right) R}-{n}_{\left(AL\right) M}\right) < 0.018$ (iib), and $- 0.014 < \left({n}_{\left(AL\right) F}-{n}_{\left(AL\right) R}\right) < 0.005$ (iiib).

### 10. Method for Producing Curable Composition for Photofabrication according to Present Disclosure

In order to produce the curable composition for photofabrication according to the present disclosure, materials exemplified in sections 3 to 8 may be used, and the respective components may be mixed such that the composition ratio of the essential components is within the above-described range, thereby obtaining a uniform composition in a liquid state, but it is necessary to satisfy the conditions shown in the feature points [4] and [5].

In the method for producing a curable composition for photofabrication according to the present disclosure, a combination satisfying the above conditions is used as a combination of the polymerizable monomer component (A), the inorganic filler (B), the photopolymerization initiator (C), the activating light absorbent (D), and the organic-inorganic composite filler (E), and the curable composition for photofabrication according to the present disclosure can be easily produced by adopting this production method.

That is, the method for producing a curable composition for photofabrication according to the present disclosure is a method for producing the curable composition for photofabrication according to the present disclosure, including a mixing step of mixing the polymerizable monomer component (A), the non-composite inorganic filler (B1), the organic-inorganic composite filler (E), the photopolymerization initiator (C), and the activating light absorbent (D). In the mixing step, as the polymerizable monomer component (A), the non-composite inorganic filler (B1), and the organic-inorganic composite filler (E), those found to satisfy all of the above-described conditions 1 to 5 and satisfy a condition (condition 6) in which a value of the light scattering index Sc (%) determined based on the intensity of the transmitted light in a specific emission angle direction obtained by measurement is 10% or less are used, the measurement being performed using a variable angle spectrophotometer in which a sample having a thickness of 0.5 mm and containing a base composition is vertically irradiated with measurement light including light having the specific wavelength λ (nm), including light having a wavelength in a range of λ ± 50 (nm) as a main component, and having a maximum intensity in the range, the base composition being a composition consisting of the polymerizable monomer component (A), the non-composite inorganic filler (B1), and the organic-inorganic composite filler (E) and having a composition ratio of the components same as a composition ratio of the curable composition for photofabrication as a production target.

The components are preferably mixed using a stirrer under light shielding of light for activating the photopolymerization initiator, for example, under red light, until the components become uniform at a room temperature, and preferably subjected to a defoaming treatment after the mixing.

### 11. Method for Producing Three-dimensional Photofabricated Product and Method for Producing Dental Restoration according to Present Disclosure

In the method for producing a three-dimensional photofabricated product according to the present disclosure, the curable composition for photofabrication according to the present disclosure is used as a liquid photocurable composition to be supplied to a vat of a vat photopolymerization device in a method for producing a three-dimensional fabricated product by the above-described vat photopolymerization process including the shaping step, the washing step, and the secondary curing step. In the method for producing a three-dimensional photofabricated product according to the present disclosure, the curable composition for photofabrication according to the present disclosure is used, and thus a three-dimensional photofabricated product having high mechanical strength and having no cracks on the actual surface can be produced.

In the method for producing a three-dimensional photofabricated product according to the present disclosure, it is preferable that the shaping step includes a first step of irradiating, with activating light, a predetermined position of the liquid photocurable composition held in the vat based on two-dimensional shape data of a height at a first ordinating order and curing the predetermined position, thereby forming a fabricated layer having a shape corresponding to the two-dimensional shape data, and using the fabricated layer as a layer to be bonded, a second step of moving the layer to be bonded upward or downward to supply the liquid photocurable composition directly above or directly below the layer to be bonded in the vat,
a third step of irradiating, with activating light, a predetermined position of the liquid photocurable composition supplied directly above or directly below the layer to be bonded, based on two-dimensional shape data of a height at an order next to the ordinating order in the previous step and curing the predetermined position, thereby forming a new fabricated layer having a shape corresponding to the two-dimensional shape data, and bonding the new fabricated layer to the layer to be bonded to obtain a stack having the new fabricated layer as a new layer to be bonded, and a fourth step of moving the stack upward or downward to supply the liquid photocurable composition directly above or directly below the new layer to be bonded in the vat,
and that a cycle including the third step and the fourth step is repeated using the new layer to be bonded as the layer to be bonded in the third step, thereby forming a new fabricated layer based on two-dimensional shape data of a height at a final ordinating order in a final third step to obtain a stack.

The vat photopolymerization process including such a shaping step can be suitably performed using a commercially available vat photopolymerization device called a 3D printer.

In the method for producing a three-dimensional photofabricated product according to the present disclosure, after the shaping step, the obtained stack is washed using an organic solvent (subjected to washing step), and then secondarily cured by performing additional activating light irradiation or a heat treatment, or both of the additional activating light irradiation and the heat treatment (subjected to secondary curing step).

Examples of the organic solvent to be used in the washing step include an alcohol solvent such as ethanol, methanol, or isopropyl alcohol; a ketone solvent such as acetone and methyl ethyl ketone; an ether solvent such as diethyl ether, diisopropyl ether, tripropylene glycol monomethyl ether, or tetrahydrofuran; an amide solvent such as N-methylpyrrolidone or dimethylacetamide; and a halogen solvent such as methylene chloride or chloroform. Among them, an alcohol solvent and an ether solvent are preferred because of high washing efficiency, and an alcohol solvent is more preferred because of low environmental load.

An irradiation wavelength when the additional activating light irradiation is performed in the secondary curing step is not particularly limited as long as activating light having the wavelength is absorbed by a photopolymerization initiator remaining in the stack to generate a radical. An irradiation intensity during the additional activating light irradiation is preferably 5 mW/cm² or more, more preferably 10 mW/cm² or more, and further preferably 30 mW/cm² or more in order for the photopolymerization initiator remaining in the stack to generate a sufficient amount of radicals. An irradiation time is not particularly limited, and is preferably 1 minute or longer, more preferably 3 minutes or longer, and further preferably 5 minutes or longer. An excessively high irradiation intensity during the additional activating light irradiation leads to excessive heating of the fabricated product, which may cause cracks in the fabricated product. Therefore, the irradiation intensity is preferably 10,000 mW/cm² or less.

When a thermal polymerization initiator is blended in the curable composition for photofabrication according to the present disclosure, the thermal polymerization initiator can be used for secondary curing by heating. A heating temperature in this case is preferably 45°C to 120°C, more preferably 50°C to 90°C, and further preferably 55°C to 80°C.

In the method for producing a dental restoration according to the present disclosure, a dental restoration such as an inlay, an onlay, a crown, or a denture is produced by the method for producing a three-dimensional photofabricated product according to the present disclosure. When such a dental restoration is to be produced, as three-dimensional shape data indicating a shape of the dental restoration (three-dimensional object) to be used in the shaping step, CAD data designed based on digital data obtained by scanning an oral cavity shape of an individual patient or an oral cavity model prepared for each individual patient may be used. With the method for producing a dental restoration according to the present disclosure, a dental restoration having high mechanical strength and having no cracks on the actual surface can be produced.

### EXAMPLES

Hereinafter, the present invention is further specifically described with reference to Examples and Comparative Examples, but the present invention is not limited to these Examples.

Compounds used below and abbreviations thereof are shown below.

### (1) Polymerizable Monomer Component (A)

### (Monomer Compound)

·UDMA: Urethane dimethacrylate
·3G: triethylene glycol dimethacrylate
·D-2.6E: bisphenol A ethylene glycol (EO) adduct dimethacrylate (EO addition number: average 2.6)
·ACMO: acryloylmorpholine
·8FHD: octafluorooctanediol dimethacrylate

### (Monomer Composition)

As a monomer composition, monomer compositions A1 to A15 prepared by mixing the monomer compounds in the compositions shown in Table 1 below were used. Numerical values in parentheses in Table 1 below indicate a content ratio (mass%) of each monomer compound.

**[Table 1]**

| | Monomer composition | n_{(D)M} | n_{(AL)M} |
|---|---|---|---|
| A1 | D-2.6E(30)/UDMA(50)/3G(20) | 1.496 | 1.539 |
| A2 | D-2.6E(50)/UDMA(36)/3G(14) | 1.508 | 1.551 |
| A3 | D-2.6E(60)/UDMA(29)/3G(11) | 1.515 | 1.558 |
| A4 | D-2.6E(70)/UDMA(21.5)/3G(8.5) | 1.521 | 1.564 |
| A5 | UDMA(70)/3G(30) | 1.478 | 1.521 |
| A6 | UDMA(70)/ACMO(30) | 1.481 | 1.524 |
| A7 | D-2.6E(38)/UDMA(35)/3G(27) | 1.499 | 1.542 |
| A8 | D-2.6E(45)/UDMA(30)/3G(25) | 1.503 | 1.546 |
| A9 | D-2.6E(50)/UDMA(25)/3G(25) | 1.506 | 1.549 |
| A10 | D-2.6E(60)/UDMA(20)/3G(20) | 1.513 | 1.556 |
| A11 | D-2.6E(58)/UDMA(21)/3G(21) | 1.511 | 1.554 |
| A12 | D-2.6E(69)/UDMA(7)/3G(24) | 1.517 | 1.560 |
| A13 | D-2.6E(78)/UDMA(5)/3G(17) | 1.524 | 1.567 |
| A14 | D-2.6E(89)/UDMA(11) | 1.534 | 1.577 |
| A15 | D-2.6E(21)/UDMA(45)/3G(34) | 1.487 | 1.530 |

A refractive index n_{(D)M} of each monomer composition with respect to a line D at 25°C and a refractive index n_{(AL)M} of each monomer composition with respect to activating light having a wavelength of 405 nm at 25°C are values measured for each sample by placing the sample on a prism of a digital Abbe refractometer (DR-M2, manufactured by Atago Co., Ltd.) and then viewing the sample from an eyepiece using an interference filter that selectively transmits measurement light (line D or light having wavelength of 405 nm), and reading a value of a display section when an intersection of a boundary line and a cross line was matched.

### (2) Inorganic Filler (B)

Inorganic fillers B-1 to B-8 obtained by mixing inorganic particulate matters shown below as they were or a plurality of types thereof were used so as to obtain the composition shown in Table 2.
·SZ-1: spherical silica-zirconia (γ-methacryloyloxypropyltrimethoxysilane surface-treated product) having an average primary particle diameter of 280 nm, a refractive index n_{(D)F} = 1.522, and n_{(AL)F} = 1.559
·SZ-2: spherical silica-zirconia (γ-methacryloyloxypropyltrimethoxysilane surface-treated product) having an average primary particle diameter of 150 nm, a refractive index n_{(D)F} = 1.522, and n_{(AL)F} = 1.559
·SZ-3: spherical silica-zirconia (γ-methacryloyloxypropyltrimethoxysilane surface-treated product) having an average primary particle diameter of 450 nm, a refractive index n_{(D)F} = 1.540, and n_{(AL)F} = 1.578
·SB-1: amorphous barium glass filler (GM27884 manufactured by Schott AG, γ-methacryloyloxypropyltrimethoxysilane surface-treated product) having an average primary particle diameter of 700 nm, a refractive index n_{(D)F} = 1.530, and n_{(AL)F} = 1.569
·SB-2: amorphous barium glass filler (GM27884 manufactured by Schott AG, γ-methacryloyloxypropyltrimethoxysilane surface-treated product) having an average primary particle diameter of 200 nm, a refractive index n_{(D)F} = 1.530, and n_{(AL)F} = 1.569
·SO-1: spherical silica (γ-methacryloyloxypropyltrimethoxysilane surface-treated product) having an average primary particle diameter of 1,000 nm, a refractive index n_{(D)F} = 1.450, and n_{(AL)F} = 1.496

The refractive indexes n_{(D)F} and n_{(AL)F} of each inorganic particulate matter are values measured by the following method. That is, ethanol or toluene and bromonaphthalene were mixed to prepare solutions having refractive indexes different from each other by 0.001, then an inorganic particulate matter mixed solution in which inorganic particulate matters were mixed into the respective solutions having different refractive indexes was prepared, and the mixture was shaken, and for a solution in which the mixed solution was observed to be most transparent, a refractive index measured in the same manner as in the measurement of the refractive index of the monomer composition was defined as the refractive index n_{(D)F} of the inorganic particulate matter. In addition, for a solution in which activating light was most clearly observed when the mixed solution was irradiated with the activating light (light having wavelength of 405 nm), a refractive index measured in the same manner as in the measurement of the refractive index of the monomer composition was defined as the refractive index n_{(AL)F} of the inorganic particulate matter.

In addition, a content ratio of particles having a particle diameter of 0.05 µm to 5.0 µm in all particles constituting each inorganic filler was measured based on the particle size distribution obtained by a microscopy method using a scanning microscope (represented as "content ratio of specific particle" in Table 2). Further, regarding each inorganic filler, a content ratio of particles having a (primary) particle diameter in which the particle diameter parameter α (represented as "content ratio of α-satisfied particle" in Table 2) is within a specific range was determined based on the particle size distribution determined by the microscopy method using a scanning microscope. Specifically, a content ratio R1 (%) of particles having the particle diameter parameter α in the range of 0.7 to 4.0, a content ratio R2 (%) of particles having the particle diameter parameter α in the range of 1.0 to 3.0, and a content ratio R3 (%) of particles having the particle diameter parameter α in the range of 1.8 to 2.8 were obtained. Results are also shown in Table 2. The particle size distribution was obtained by measuring the number n (50 or more) of all particles observed in a unit field of view of a scanning electron microscope photograph of each inorganic filler and the primary particle diameter (maximum diameter) Xᵢ (nm) of all particles.

**[Table 2]**

| | Composition of inorganic particulate matter | Content ratio of specific particle (%) | Content ratio of α-satisfied particle | | |
|---|---|---|---|---|---|
| | Inorganic particulate matter: content ratio (mass%) | | α = 0.7-4.0 R1 (%) | α = 1.0-3.0 R2 (%) | α = 1.8-2.8 R3 (%) |
| B-1 | SZ-1:100 | 100 | 100 | 100 | 100 |
| B-2 | SZ-2:100 | 100 | 100 | 80 | 30 |
| B-3 | SZ-3:100 | 100 | 95 | 50 | 20 |
| B-4 | SZ-1: 80 | 100 | 90 | 85 | 83 |
| | SB-1: 20 | | | | |
| B-5 | SB-1: 80 | 100 | 45 | 30 | 20 |
| | SB-2: 20 | | | | |
| B-6 | SZ-3: 70 | 100 | 97 | 50 | 40 |
| | SZ-1: 30 | | | | |
| B-7 | SZ-1: 70 | 100 | 98 | 95 | 92 |
| | SO-1: 30 | | | | |
| B-8 | SO-1: 100 | 100 | 0 | 0 | 0 |

### (3) Organic-inorganic Composite Filler

Organic-inorganic composite fillers CF1 to CF9 were prepared in the same manner as in the method described in Example 1 of Patent Document 4 (PCT International Publication No. WO2013/039169), except that inorganic particulate matters shown in Table 3 below and a monomer composition having a composition shown in Table 3 below were used as the polymerizable monomer component serving as a raw material of the composite inorganic filler (B2) and the resin (R) that constitute the organic-inorganic composite filler. Numerical values in parentheses in Table 3 below indicate a content ratio (mass%) of each monomer compound. In organic-inorganic composite particles constituting the organic-inorganic composite fillers, a cured product of the monomer composition is the resin (R), and the resin (R) covers a surface of inorganic primary particles serving as the composite inorganic filler (B2) and is a composite in which the inorganic primary particles are bonded to each other.

A refractive index n_{(AL)R} in Table 3 is a value measured by placing a cured product of the monomer composition having a thickness of 0.1 mm on a prism of a digital Abbe refractometer (DR-M2, manufactured by Atago Co., Ltd.) and then viewing a sample from an eyepiece using an interference filter that selectively transmits measurement light (light having wavelength of 405 nm), and reading a value of a display section when an intersection of a boundary line and a cross line was matched.

**[Table 3]**

| | Composite inorganic filler (B2) | Refractive index n_{(AL)F} | Raw material monomer composition of resin (R) | Refractive index of resin n_{(AL)R} |
|---|---|---|---|---|
| CF1 | SZ-1 | 1.559 | UDMA(100) | 1.550 |
| CF2 | SZ-1 | 1.559 | UDMA(85)/8FHD(15) | 1.542 |
| CF3 | SZ-1 | 1.559 | UDMA(90)/D-2.6E(10) | 1.555 |
| CF4 | SZ-1 | 1.559 | UDMA(80)/D-2.6E(20) | 1.561 |
| CF5 | SZ-1 | 1.559 | UDMA(60)/D-2.6E(40) | 1.571 |
| CF6 | SZ-1 | 1.559 | UDMA(75)/8FHD(25) | 1.535 |

### (4) Photopolymerization Initiator

·BAPO: phenyl bis(2,4,6-trimethylbenzoyl)phosphine oxide (radicals are generated by irradiation with activating light having wavelength of 405 nm)
·TPO: 2,4,6-trimethylbenzoyl-diphenylphosphine oxide
·CQ: camphorquinone
·DMBE: dimethyl p-ethoxybenzoate

### (5) Activating Light Absorbent

·SS3: 2-(3-tert-butyl-2-hydroxy-5-methylphenyl)-5-chloro-2H-benzotriazole

### (6) Polymerization Inhibitor

·BHT: dibutylhydroxytoluene
·HQME: hydroquinone methyl ether

### (7) Coloring Substance

·Titanium oxide (average primary particle diameter: 200 µm)

### I. Reference Examples and Reference Comparative Examples

As described above, the curable composition for photofabrication according to the present disclosure belongs to the category of the proposed curable composition. The proposed curable composition exhibits an effect of being able to produce a three-dimensional photofabricated product having a low risk of a decrease in fluidity and a low risk of sedimentation of an inorganic filler, and having excellent mechanical strength and good shape accuracy while suppressing generation of cracks on a surface. Therefore, this point will be described first with reference examples and reference comparative examples.

### <Reference Example 1>

### (1) Preparation of Base Composition for Sc Evaluation and Curable Composition for Photofabrication

To 100 parts by mass of a polymerizable monomer component containing a monomer composition A1 (50 parts by mass of UDMA, 20 parts by mass of 3G, 30 parts by mass of D-2.6E), 150 parts by mass of B-1 as a non-composite inorganic filler (B1) was added, and the mixture was stirred and mixed until the mixture became uniform, followed by defoaming to prepare a base composition for Sc evaluation.

In addition, a base composition for raw materials is separately prepared in the same manner, 1.4 parts by mass of BAPO as a photopolymerization initiator, 0.7 parts by mass of SS3 as an activating light absorbent, and a total of 0.2 parts by mass of a polymerization inhibitor containing 0.1 parts by mass of HQME and 0.1 parts by mass of BHT (blending amounts are blending amounts with respect to 100 parts by mass of polymerizable monomer component) were added therein, and the mixture was stirred and mixed under red light until the mixture became uniform, followed by defoaming to prepare a liquid curable composition for photofabrication.

### (2) Measurement of Light Scattering Index Sc

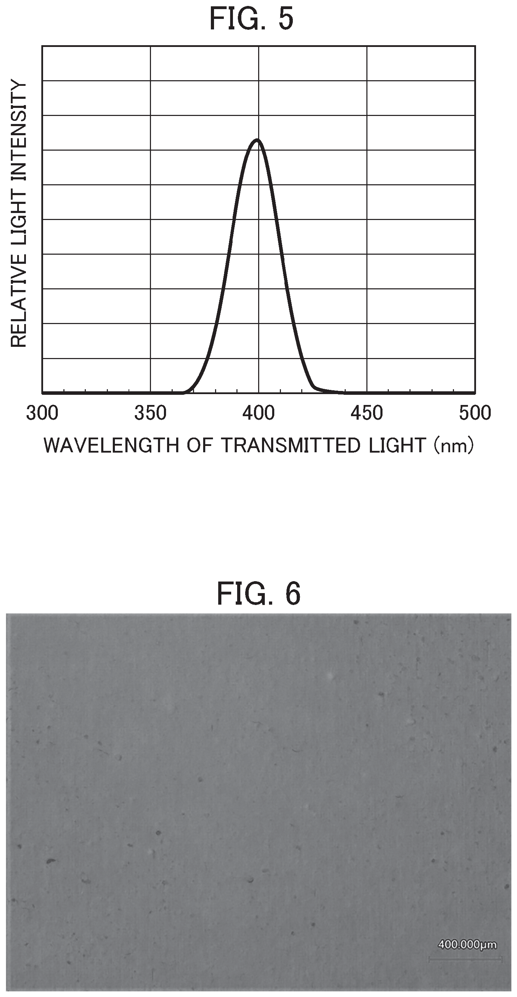

The base composition for Sc evaluation was filled in a mold (25 mm × 25 mm × thickness of 0.5 mm) made of PTFE. Upper and lower surfaces were pressed with a slide glass to adjust the thickness of the composition to 0.5 mm, and then the slide glass was removed to prepare an evaluation sample. This evaluation sample was set in a three-dimensional variable angle spectrophotometer (GP-200, manufactured by Murakami Color Research Laboratory Co., Ltd.), and was vertically irradiated with measurement light to measure a luminous intensity distribution of transmitted light. In the measurement, an interference filter (GP-200 manufactured by Murakami Color Technology Research Co., Ltd.) was installed between a light source of the three-dimensional variable angle spectrophotometer and the evaluation sample, and light having a wavelength distribution (relative spectral distribution) illustrated in Fig. 5 was used as measurement light. The light scattering index (Sc) was calculated in accordance with the following formula based on the obtained luminous intensity distribution. $Sc = \left\{\left({I}_{70}+{I}_{75}+{I}_{80}\right)/\left({I}_{0}\times 3\right)\right\} \times 100$ As a result, the light scattering index Sc was 0.1%.

### (3) Measurement of Transmittance to Activating Light

The curable resin composition for photofabrication obtained in (1) above was filled in a mold (25 mm × 25 mm × thickness of 0.5 mm) made of a PTFE resin. Upper and lower surfaces were pressed with a slide glass to adjust the thickness of the composition to 0.5 mm, and then the slide glass was removed to prepare a resin composition having a thickness of 0.5 mm. Regarding the resin composition, a transmittance to light having a wavelength of 405 nm was measured using a color difference meter (spectrophotometer SE7700 manufactured by NIPPON DENSHOKU KOGYO CO., LTD.). As a result, the transmittance was 5.3%.

### (4) Production of Three-dimensional Fabricated Product

The curable composition for photofabrication obtained in (1) above was supplied to a resin tray (vat) of a 3D printer (DW029D manufactured by DWS, wavelength: 405 nm, irradiation intensity: 83 mW), and a shaping step was performed using stereolithography data (hereinafter, abbreviated as "stl data") of a rectangular parallelepiped shape of 10 mm × 10 mm × 25 mm to prepare a shaped product (stack) having a stacked structure (made of cured product of curable composition for photofabrication). Next, the resulting shaped product was then immersed in a plastic container filled with ethanol for 15 minutes, shaken gently to perform a washing step, then dried, and subjected to additional light irradiation (secondary curing) for 30 minutes using a UV CURING UNIT UVIS-2 (manufactured by DWS) to produce a three-dimensional photofabricated product.

### (5) Evaluation of Three-dimensional Photofabricated Product

The three-dimensional photofabricated product obtained in (4) above was used as an evaluation sample for fabrication accuracy evaluation, crack evaluation, and 3-point bending fracture strength evaluation. Evaluation methods and evaluation results are described below.

### (Fabrication Accuracy Evaluation)

A length of each side of the evaluation sample was measured in 0.01 mm increments, a value ("measured value/stl data set value") obtained by dividing a measured value by a set value at the time of shaping (length of each side set in stl data) was calculated for each side, and an average value thereof was evaluated as an average value of fabrication accuracy. As a result, the average value of the fabrication accuracy was "1.01".

### (Crack Evaluation)

A surface of the evaluation sample was observed with an optical microscope (50X), the evaluation sample was coated with platinum to a thickness of 5 nm and then observed with a scanning microscope (1,000X) to check for the number of cracks observed on one surface of 10 mm × 25 mm in the evaluation sample and a crack width, and evaluation was performed according to the following evaluation criteria. As a result, a result of the crack evaluation was "A1".

### -Evaluation Criteria-

A0: No cracks are observed on the surface of the cured product.
A1: The number of cracks observed on the surface of the cured product is 5 or less, and each crack width is 10 µm or less, which is within an allowable range.
A2: The number of cracks observed on the surface of the cured product is 10 or less, and each crack width is 10 µm or less, which is within the allowable range.
A3: The number of cracks observed on the surface of the cured product is 20 or less, and each crack width is 10 µm or less, which is within the allowable range.
B: The number of cracks observed on the surface of the cured product is 20 or less, and the crack width is 10 µm to 40 µm.
C: 20 or more cracks having a crack width of 10 µm to 40 µm are observed on the surface of the cured product.
D: A large amount of cracks having a crack width of 40 µm or more are observed on the surface of the cured product.

### <Reference Examples 2 to 14 and Reference Comparative Examples 1 to 8>

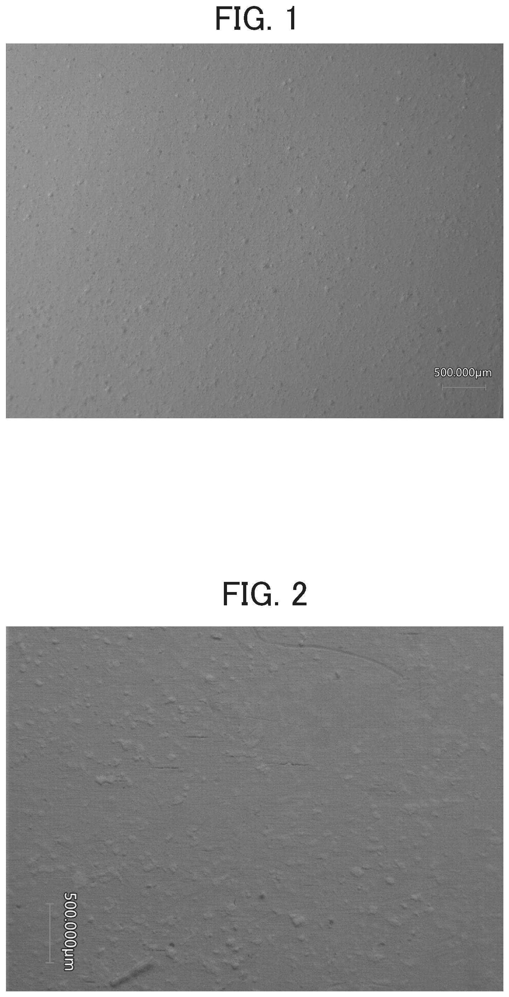

Base compositions for Sc evaluation and curable compositions for photofabrication were prepared in the same manner as in Reference Example 1 except that respective components and blending amounts thereof used in preparing the base composition for Sc evaluation and the curable composition for photofabrication in Reference Example 1 were changed as shown in Tables 4 and 5. Thereafter, each of the obtained compositions was evaluated in the same manner as in Reference Example 1, and a three-dimensional photofabricated product using the obtained curable composition for photofabrication was produced and evaluated in the same manner as in Reference Example 1. Evaluation results are shown in Tables 6 and 7. In addition, optical microscope images (magnification of 50X) of Reference Example 7 (evaluated as A0), Reference Example 11 (evaluated as A2), and Reference Comparative Example 1 (evaluated as D), which are taken during observation at the time of crack evaluation, are shown in Figs. 1, 2, and 3, respectively.

### <Reference Comparative Example 9>

A curable composition for photofabrication was prepared according to the method described in Example 1 of Patent Document 3. Specifically, the polymerizable monomer component (A) and the inorganic filler (B) of the types shown in Table 5 are used in blending ratios shown in Table 5, and 3.0 parts by mass of a polymerization initiator containing TPO and 0.05 parts by mass of a polymerization inhibitor containing BHT (blending amounts thereof are each blending amount with respect to 100 parts by mass of polymerizable monomer component) were added to prepare a curable composition for photofabrication. A three-dimensional photofabricated product using the obtained curable composition for fabrication was produced and evaluated in the same manner as in Reference Example 1. In addition, a base composition for Sc evaluation and a non-cured state composition (for transmittance evaluation) were prepared in the same manner as in Reference Example 1, and each composition was evaluated in the same manner as in Reference Example 1. Evaluation results are shown in Table 7. In addition, optical microscope images (magnification of 50X) taken during observation at the time of crack evaluation in Reference Comparative Example 9 (evaluated as C) are shown in Fig. 4.

**[Table 4]**

| No. | | Curable composition for photofabrication abbreviation of composition component (parts by mass) | | | | | |
|---|---|---|---|---|---|---|---|
| | | Polymeriza ble monomer component (A) | Non-composite inorganic filler (B1) | Photopolyme rization initiator (C) | Activating light absorbent (D) | Other components polymerization inhibitor | Other Components coloring substance |
| | 1 | A1(100) | B-1(150) | BAPO(1.4) | SS3(0.7) | BHT(0.1) | - |
| | | | | | | HQME(0.1) | |
| | 2 | A1(100) | B-1(100) | BAPO(1.4) | SS3(0.7) | BHT(0.1) | - |
| | | | | | | HQME(0.1) | |
| | 3 | A1(100) | B-1(67) | BAPO(1.4) | SS3(0.7) | BHT(0.1) | - |
| | | | | | | HQME(0.1) | |
| | 4 | A2(100) | B-1(150) | BAPO(1.4) | SS3(0.7) | BHT(0.1) | - |
| | | | | | | HQME(0.1) | |
| | 5 | A3(100) | B-1(150) | BAPO(1.4) | SS3(0.7) | BHT(0.1) | - |
| | | | | | | HQME(0.1) | |
| | 6 | A4(100) | B-1(150) | BAPO(1.4) | SS3(0.7) | BHT(0.1) | - |
| | | | | | | HQME(0.1) | |
| Referen ce Example | 7 | A4(100) | B-1(150) | BAPO(1.4) | SS3(0.3) | BHT(0.1) | - |
| | | | | | | HQME(0.1) | |
| | 8 | A4(100) | B-1(150) | BAPO(1.4) | SS3(0.1) | BHT(0.1) | - |
| | | | | | | HQME(0.1) | |
| | 9 | A4(100) | B-1(150) | BAPO(1.4) | - | BHT(0.1) | - |
| | | | | | | HQME(0.2) | |
| | 10 | A4(100) | B-1(150) | BAPO(1.4) | SS3(0.7) | BHT(0.1) | Titanium oxide(0.02) |
| | | | | | | HQME(0.1) | |
| | 11 | A4(100) | B-1(150) | BAPO(1.4) | SS3(0.7) | BHT(0.1) | Titanium oxide(0.04) |
| | | | | | | HQME(0.1) | |
| | 12 | A1(100) | B-2(150) | BAPO(1.4) | SS3(0.7) | BHT(0.1) | - |
| | | | | | | HQME(0.1) | |
| | 13 | A1(100) | B-4(150) | BAPO(1.4) | SS3(0.7) | BHT(0.1) | - |
| | | | | | | HQME(0.1) | |
| | 14 | A1(100) | B-5(150) | BAPO(1.4) | SS3(0.7) | BHT(0.1) | - |
| | | | | | | HQME(0.1) | |

**[Table 5]**

| No. | | Curable composition for photofabrication abbreviation of composition component (parts by mass) | | | | | |
|---|---|---|---|---|---|---|---|
| | | Polymeriza ble monomer component (A) | Non-composite inorganic filler (B1) | Photopolyme rization initiator (C) | Activating light absorbent (D) | Other components polymerization inhibitor | Other Components coloring substance |
| | 1 | A1(100) | B-3(150) | BAPO(1.4) | SS3(0.7) | BHT(0.1) | - |
| | | | | | | HQME(0.1) | |
| | 2 | A1(100) | B-3(150) | BAPO(1.4) | SS3(0.3) | BHT(0.1) | - |
| | | | | | | HQME(0.1) | |
| | 3 | A1(100) | B-3(150) | BAPO(1.4) | SS3(0.1) | BHT(0.1) | - |
| | | | | | | HQME(0.1) | |
| Referen ce Compara tive Example | 4 | A4(100) | B-6(150) | BAPO(1.4) | SS3(0.7) | BHT(0.1) | - |
| | | | | | | HQME(0.1) | |
| | 5 | A1(100) | B-7(150) | BAPO(1.4) | SS3(0.7) | BHT(0.1) | - |
| | | | | | | HQME(0.1) | |
| | 6 | A5(100) | B-1(150) | BAPO(1.4) | SS3(0.7) | BHT(0.1) | - |
| | | | | | | HQME(0.1) | |
| | 7 | A5(100) | B-2(150) | BAPO(1.4) | SS3(0.7) | BHT(0.1) | - |
| | | | | | | HQME(0.1) | |
| | 8 | A1(100) | B-3(67) | BAPO(1.4) | SS3(0.7) | BHT(0.1) | - |
| | | | | | | HQME(0.1) | |
| | 9 | A6(100) | B-8(100) | TPO(3.0) | - | BHT(0.05) | - |

**[Table 6]**

| No. | | Difference in refractive indexes of (A) and (B1) \|n(D)F - n(D)M\| | Transmittance to activating light (405 nm) (%) | Light scattering index Sc of base composition (%) | Evaluation results of photofabricated product | |
|---|---|---|---|---|---|---|
| | | | | | Fabrication accuracy | Crack evaluation |
| | 1 | 0.026 | 5.3 | 0.1 | 1.01 | A1 |
| | 2 | 0.026 | 5.5 | 0.1 | 1.01 | A1 |
| | 3 | 0.026 | 5.7 | 0 | 1.01 | A1 |
| | 4 | 0.007 | 9.4 | 0 | 1.01 | A1 |
| | 5 | 0.014 | 7.0 | 0 | 1.01 | A1 |
| | 6 | 0.001 | 14.2 | 0 | 1.01 | A1 |
| Referen ce Example | 7 | 0.001 | 18.8 | 0 | 1.02 | A0 |
| | 8 | 0.001 | 25.1 | 0 | 1.05 | A0 |
| | 9 | 0.001 | 30.2 | 0 | 1.20 | A0 |
| | 10 | 0.001 | 8.2 | 0.2 | 1.01 | A2 |
| | 11 | 0.001 | 5.5 | 0.4 | 1.01 | A2 |
| | 12 | 0.026 | 3.1 | 2.9 | 1.02 | A3 |
| | 13 | 0.034 | 3.1 | 0.1 | 1.02 | A3 |
| | 14 | 0.034 | 2.6 | 0.1 | 1.02 | A3 |

**[Table 7]**

| No. | | Difference in refractive indexes of (A) and (B1) \|n(D)F - n(D)M\| | Transmittance to activating light (405 nm) (%) | Light scattering index Sc of base composition (%) | Evaluation results of photofabricated product | |
|---|---|---|---|---|---|---|
| | | | | | Fabrication accuracy | Crack evaluation |
| | 1 | 0.040 | 0.2 | 26 | 1.06 | D |
| | 2 | 0.040 | 0.4 | 26 | 1.20 | D |
| Referen ce Compara tive Example | 3 | 0.040 | 0.9 | 26 | 1.50 | B |
| | 4 | 0.019 | 0.49 | 9.3 | 1.05 | C |
| | 5 | 0.046 | 0.51 | 1.1 | 1.02 | D |
| | 6 | 0.044 | 0.44 | 0.7 | 1.02 | D |
| | 7 | 0.044 | 0.33 | 4.2 | 1.02 | D |
| | 8 | 0.040 | 0.97 | 12.2 | 1.05 | C |
| | 9 | 0.409 | 0.50 | 15.2 | 1.01 | C |

As shown in Table 6, since the curable compositions for photofabrication of Reference Examples 1 to 14 have a small light scattering index Sc and a high activating light transmittance, the fabrication accuracy is high and the generation of cracks is suppressed.

On the other hand, as shown in Table 7, since the curable compositions for photofabrication of Reference Comparative Examples 1 to 3 have a large light scattering index Sc and a small activating light transmittance, the fabrication accuracy is low and cracks are generated. In addition, in the curable compositions for photofabrication of Reference Comparative Examples 4 to 7, although the light scattering index Sc is small, since the activating light transmittance is small, cracks are generated. Since the curable composition for photofabrication of Reference Comparative Example 8 has a large light scattering index Sc, the fabrication accuracy is low and cracks are generated. The curable composition for photofabrication of Reference Comparative Example 9 is prepared according to the method described in Patent Document 3, since the activating light transmittance is small, cracks are generated.

### II. Examples and Comparative Examples

Examples and Comparative Examples of the curable composition for photofabrication according to the present disclosure are shown below.

### <Example 1>

### (1) Preparation of Base Composition for Sc Evaluation and Photocurable Composition

To 100 parts by mass of a polymerizable monomer component (A) containing a monomer composition A7 (35 parts by mass of UDMA, 27 parts by mass of 3G, 38 parts by mass of D-2.6E), 60 parts by mass of B-1 as a non-composite inorganic filler (B1) and 90 parts by mass of CF1 as an organic-inorganic composite filler (E) was added, and the mixture was stirred and mixed until the mixture became uniform, followed by defoaming to prepare a base composition for Sc evaluation.

In addition, a base composition for raw materials is separately prepared in the same manner, 1.4 parts by mass of BAPO as a photopolymerization initiator, 0.7 parts by mass of SS3 as an activating light absorbent, and a total of 0.2 parts by mass of a polymerization inhibitor containing 0.1 parts by mass of HQME and 0.1 parts by mass of BHT (blending amounts are blending amounts with respect to 100 parts by mass of polymerizable monomer component) were added therein, and the mixture was stirred and mixed under red light until the mixture became uniform, followed by defoaming to prepare a liquid curable composition for photofabrication.

### (Measurement of Light Scattering Index Sc and Transmittance to Activating Light)

For the obtained base composition for Sc evaluation and the curable composition for photofabrication, the light scattering index Sc and the transmittance to activating light were measured in the same manner as in Reference Example 1. As a result, the light scattering index Sc was 0.5%, and the transmittance to activating light was 6.9%.

### (Evaluation of Three-dimensional Photofabricated Product and Crack Evaluation)

A three-dimensional photofabricated product was produced in the same manner as in Reference Example 1 using the obtained curable composition for photofabrication, and the obtained three-dimensional photofabricated product was evaluated. As a result, an average value of the fabrication accuracy was "1.01", and a result of the crack evaluation was "A1". An optical microscope photograph (magnification of 50X) taken during observation at the time of crack evaluation is shown in Fig. 6.

### (Evaluation of Fluidity)

500 g of the curable composition for photofabrication obtained in Example 1 was supplied to a resin tray (vat) of a 3D printer (DW029D manufactured by DWS, wavelength: 405 nm, irradiation intensity: 83 mW), and a shaping step was performed using stereolithography data (stl data) of a rectangular parallelepiped shape of 150 mm × 150 mm × 200 mm to prepare a shaped product (stack) having a stacked structure (made of cured product of curable composition for photofabrication). Next, the resulting shaped product was then immersed in a plastic container filled with ethanol for 15 minutes, shaken gently to perform a washing step, then dried, and subjected to additional light irradiation (secondary curing) for 30 minutes using a UV CURING UNIT UVIS-2 (manufactured by DWS) to produce a three-dimensional photofabricated product.

The obtained rectangular parallelepiped three-dimensional photofabricated product having a size of 150 mm × 150 mm × 200 mm was visually observed and observed with an optical microscope (50X) to evaluate whether there was a defective portion in the rectangular parallelepiped shape. As a result, it was confirmed that no defective portion is observed by either the visual observation or the optical microscope (50X), and the fluidity is high (evaluation result: A).

### <Examples 2 to 20 and Comparative Examples 1 to 6>

Base compositions for Sc evaluation and curable compositions for photofabrication were prepared in the same manner as in Example 1 except that respective components and blending amounts thereof used in preparing the base composition for Sc evaluation and the curable composition for photofabrication in Example 1 were changed as shown in Tables 8 and 9. Thereafter, each of the obtained compositions was evaluated in the same manner as in Example 1, and a three-dimensional photofabricated product using the obtained curable composition for photofabrication was produced and evaluated in the same manner as in Example 1. Evaluation results are shown in Tables 10 and 11.

**[Table 8]**

| No. | | Curable composition for photofabrication abbreviation of composition component (parts by mass) | | | | | |
|---|---|---|---|---|---|---|---|
| | | Polymeriza ble monomer component (A) | Non-composite inorganic filler (B1) | Photopolyme rization initiator (C) | Activating light absorbent (D) | Organic-inorganic composite filler (E) | Other components polymerization inhibitor |
| | 1 | A7(100) | B-1(60) | BAPO(1.4) | SS3(0.7) | CF1(90) | BHT(0.1) |
| | | | | | | | HQME(0.1) |
| | 2 | A8(100) | B-1(60) | BAPO(1.4) | SS3(0.7) | CF1(90) | BHT(0.1) |
| | | | | | | | HQME(0.1) |
| | 3 | A9(100) | B-1(60) | BAPO(1.4) | SS3(0.7) | CF1(90) | BHT(0.1) |
| | | | | | | | HQME(0.1) |
| | 4 | A10(100) | B-1(60) | BAPO(1.4) | SS3(0.7) | CF1(90) | BHT(0.1) |
| | | | | | | | HQME(0.1) |
| | 7 | A7(100) | B-1(60) | BAPO(1.4) | SS3(0.7) | CF2(90) | BHT(0.1) |
| | | | | | | | HQME(0.1) |
| | 8 | A8(100) | B-1(60) | BAPO(1.4) | SS3(0.7) | CF2(90) | BHT(0.1) |
| | | | | | | | HQME(0.1) |
| | 9 | A9(100) | B-1(60) | BAPO(1.4) | SS3(0.7) | CF2(90) | BHT(0.1) |
| | | | | | | | HQME(0.2) |
| | 10 | A10(100) | B-1(60) | BAPO(1.4) | SS3(0.7) | CF2(90) | BHT(0.1) |
| | | | | | | | HQME(0.1) |
| | 11 | A1(100) | B-1(60) | BAPO(1.4) | SS3(0.7) | CF4(90) | BHT(0.1) |
| Example | | | | | | | HQME(0.1) |
| | 12 | A7(100) | B-1(60) | BAPO(1.4) | SS3(0.7) | CF4(90) | BHT(0.1) |
| | | | | | | | HQME(0.1) |
| | 13 | A8(100) | B-1(60) | BAPO(1.4) | SS3(0.7) | CF4(90) | BHT(0.1) |
| | | | | | | | HQME(0.1) |
| | 14 | A11(100) | B-1(60) | BAPO(1.4) | SS3(0.7) | CF4(90) | BHT(0.1) |
| | | | | | | | HQME(0.1) |
| | 15 | A12(100) | B-1(60) | BAPO(1.4) | SS3(0.7) | CF4(90) | BHT(0.1) |
| | | | | | | | HQME(0.1) |
| | 16 | A13(100) | B-1(60) | BAPO(1.4) | SS3(0.7) | CF4(90) | BHT(0.1) |
| | | | | | | | HQME(0.1) |
| | 17 | A8(100) | B-1(60) | BAPO(1.4) | SS3(0.7) | CF5(90) | BHT(0.1) |
| | | | | | | | HQME(0.1) |
| | 18 | A11(100) | B-1(60) | BAPO(1.4) | SS3(0.7) | CF5(90) | BHT(0.1) |
| | | | | | | | HQME(0.1) |
| | 19 | A12(100) | B-1(60) | BAPO(1.4) | SS3(0.7) | CF5(90) | BHT(0.1) |
| | | | | | | | HQME(0.1) |
| | 20 | A13(100) | B-1(60) | BAPO(1.4) | SS3(0.7) | CF5(90) | BHT(0.1) |
| | | | | | | | HQME(0.1) |

**[Table 9]**

| No. | | Curable composition for photofabrication abbreviation of composition component (parts by mass) | | | | | |
|---|---|---|---|---|---|---|---|
| | | Polymeriza ble monomer component (A) | Non-composite inorganic filler (B1) | Photopolyme rization initiator (C) | Activating light absorbent (D) | Organic-inorganic composite filler (E) | Other components polymerization inhibitor |
| | 1 | A8(100) | B-1(60) | BAPO(1.4) | SS3(0.7) | CF6(90) | BHT(0.1) |
| | | | | | | | HQME(0.1) |
| | 2 | A5(100) | B-1(60) | BAPO(1.4) | SS3(0.7) | CF1(90) | BHT(0.1) |
| | | | | | | | HQME(0.1) |
| Compara tive Example | 3 | A13(100) | B-1(60) | BAPO(1.4) | SS3(0.7) | CF1(90) | BHT(0.1) |
| | | | | | | | HQME(0.1) |
| | 4 | A14(100) | B-1(60) | BAPO(1.4) | SS3(0.7) | CF1(90) | BHT(0.1) |
| | | | | | | | HQME(0.1) |
| | 5 | A15(100) | B-1(60) | BAPO(1.4) | SS3(0.7) | CF4(90) | BHT(0.1) |
| | | | | | | | HQME(0.1) |
| | 6 | A14(100) | B-1(60) | BAPO(1.4) | SS3(0.7) | CF4(90) | BHT(0.1) |
| | | | | | | | HQME(0.1) |

**[Table 10]**

| No. | | n_{(AL)F} - n_{(AL)M} | n_{(AL)R} - n_{(AL)M} | n_{(AL)F} - n_{(AL)R} | Transmittance to activating light (405 nm) (%) | Light scattering index Sc of base composition (%) | Evaluation results of photofabricated product | | Evaluation Evaluation result of fluidity |
|---|---|---|---|---|---|---|---|---|---|
| | | | | | | | Fabrication accuracy | Crack evaluation | |
| | 1 | 0.0170 | 0.0080 | 0.009 | 6.9 | 0.5 | 1.01 | A1 | A |
| | 2 | 0.0130 | 0.0040 | 0.009 | 8.3 | 0.3 | 1.01 | A1 | A |
| | 3 | 0.0100 | 0.0010 | 0.009 | 6.4 | 0.5 | 1.01 | A1 | A |
| | 4 | 0.0030 | -0.0060 | 0.009 | 3.3 | 3.6 | 1.02 | Al | A |
| | 7 | 0.0170 | 0.0000 | 0.017 | 7.3 | 0.5 | 1.01 | A1 | A |
| | 8 | 0.0130 | -0.0040 | 0.017 | 6.4 | 0.5 | 1.01 | A1 | A |
| | 9 | 0.0100 | -0.0070 | 0.017 | 5.6 | 0.6 | 1.01 | A1 | A |
| | 10 | 0.0030 | -0.0140 | 0.017 | 3.3 | 3.2 | 1.03 | A1 | A |
| Example | 11 | 0.0200 | 0.0220 | -0.002 | 3.2 | 3.4 | 1.03 | A1 | A |
| | 12 | 0.0170 | 0.0190 | -0.002 | 4.9 | 0.5 | 1.02 | A1 | A |
| | 13 | 0.0130 | 0.0150 | -0.002 | 9.5 | 0.2 | 1.01 | A1 | A |
| | 14 | 0.0050 | 0.0070 | -0.002 | 13.7 | 0.1 | 1.01 | AO | A |
| | 15 | -0.0010 | 0.0010 | -0.002 | 7.3 | 0.2 | 1.01 | A1 | A |
| | 16 | -0.0080 | -0.0060 | -0.002 | 3.3 | 3.1 | 1.02 | A1 | A |
| | 17 | 0.0130 | 0.0250 | -0.012 | 8.3 | 0.3 | 1.01 | A1 | A |
| | 18 | 0.0050 | 0.0170 | -0.012 | 13.7 | 0.1 | 1.01 | AO | A |
| | 19 | -0.0010 | 0.0110 | -0.012 | 8.3 | 0.3 | 1.01 | A1 | A |
| | 20 | -0.0080 | 0.0040 | -0.012 | 3.7 | 3.4 | 1.01 | A1 | A |

**[Table 11]**

| No. | | n_{(AL)F} - n_{(AL)M} | n_{(AL)r} - n_{(AL)M} | n_{(AL)F} - n_{(AL)R} | Transmittance to activating light (405 nm) (%) | Light scattering index Sc of base composition (%) | Evaluation results of photofabricated product | | Evaluation result of fluidity |
|---|---|---|---|---|---|---|---|---|---|
| | | | | | | | Fabrication accuracy | Crack evaluation | |
| | 1 | 0.0130 | -0.0110 | 0.024 | 2.8 | 6.2 | 1.03 | B | A |
| | 2 | 0.0380 | 0.0290 | 0.009 | 1.1 | 2.0 | 1.03 | B | A |
| Compara tive Example | 3 | -0.0080 | -0.0170 | 0.009 | 2.1 | 6.9 | 1.03 | B | A |
| | 4 | -0.0180 | -0.0270 | 0.009 | 0.5 | 6.9 | 1.04 | B | A |
| | 5 | 0.0290 | 0.0310 | -0.002 | 0.8 | 3.6 | 1.03 | B | A |
| | 6 | -0.0180 | -0.0160 | -0.002 | 1.3 | 7.7 | 1.03 | B | A |

As shown in Table 10, since the curable compositions for photofabrication of Examples 1 to 20 have a small light scattering index Sc and a high activating light transmittance, the fabrication accuracy is high and the generation of cracks is suppressed.

On the other hand, as shown in Table 11, in the curable composition for photofabrication of Comparative Example 1, since a difference in refractive index with respect to light having a wavelength of 405 nm between the composite inorganic filler (B2) and the resin (R) constituting the organic-inorganic composite filler (E) is large, and a transmittance with respect to the light having a wavelength of 405 nm is small, cracks increase. In the curable composition for photofabrication of Comparative Example 2, since a difference in refractive index with respect to light having a wavelength of 405 nm between the polymerizable monomer component (A) and the composite inorganic filler (B2) constituting the organic-inorganic composite filler (E) is large, the transmittance with respect to the light having a wavelength of 405 nm is small, and cracks increase. In the curable composition for photofabrication of Comparative Example 3, since a difference in refractive index with respect to light having a wavelength of 405 nm between the polymerizable monomer component (A) and the resin (R) constituting the organic-inorganic composite filler (E) is large, the transmittance with respect to the light having a wavelength of 405 nm is small, and cracks increase. In the curable compositions for photofabrication of Comparative Examples 4 to 6, since a difference in refractive index with respect to light having a wavelength of 405 nm between the polymerizable monomer component (A), and the composite inorganic filler (B2) and the resin (R) constituting the organic-inorganic composite filler (E) is large, the transmittance with respect to the light having a wavelength of 405 nm is small, and cracks increase.

### <Examples 21 to 32 and Comparative Examples 7 to 10>

Three-dimensional photofabricated products were produced and evaluated in the same manner as in Examples 1, 14, and 18 and Comparative Example 1 except that the 3D printer used for producing the three-dimensional photofabricated products in Examples 1, 14, and 18 and Comparative Example 1 was changed as shown in Tables 12 and 13. Evaluation results are shown in Tables 12 and 13. The evaluation results of Examples 1, 14, and 18 and Comparative Example 1 are also shown in Tables 12 and 13. Curable compositions for photofabrication used in Examples 21 to 24 have the same composition as the curable composition for photofabrication used in Example 1. Curable compositions for photofabrication used in Examples 25 to 29 have the same composition as the curable composition for photofabrication used in Example 14. Curable compositions for photofabrication used in Examples 29 to 32 have the same composition as the curable composition for photofabrication used in Example 18. Curable compositions for photofabrication used in Comparative Examples 7 to 10 have the same composition as the curable composition for photofabrication used in Comparative Example 1.

3D printers 3DP1 to 3DP5 in Tables 12 and 13 mean the following 3D printers, respectively.
·3DP1: DW029D (SLA, wavelength: 405 nm) manufactured by DWS
·3DP2: SEGA 3D (DLP, wavelength: 405 nm) manufactured by 3BFAB
·3DP3: iLux Pro Dental (DLP, wavelength: 385 nm) manufactured by Lux Creo
·3DP4: Smapri Sonic 8K (LCD, wavelength: 405 nm) manufactured by Phrozen
·3DP5: SOL plus (LCD, wavelength: 385 nm to 405 nm) manufactured by Ackuretta

**[Table 12]**

| No. | | Evaluation results of photofabricated product | | |
|---|---|---|---|---|
| | | 3D printer | Fabrication accuracy | Crack evaluation |
| | 1 | 3DP1 | 1.01 | A1 |
| | 21 | 3DP2 | 1.02 | A1 |
| | 22 | 3DP3 | 1.01 | A1 |
| | 23 | 3DP4 | 1.02 | A1 |
| | 24 | 3DP5 | 1.01 | A1 |
| | 14 | 3DP1 | 1.01 | A0 |
| | 25 | 3DP2 | 1.01 | A0 |
| Example | 26 | 3DP3 | 1.02 | A0 |
| | 27 | 3DP4 | 1.01 | A0 |
| | 28 | 3DP5 | 1.02 | A0 |
| | 18 | 3DP1 | 1.01 | A0 |
| | 29 | 3DP2 | 1.02 | A0 |
| | 30 | 3DP3 | 1.01 | A0 |
| | 31 | 3DP4 | 1.02 | A0 |
| | 32 | 3DP5 | 1.01 | A0 |

**[Table 13]**

| No. | | Evaluation results of photofabricated product | | |
|---|---|---|---|---|
| | | 3D printer | Fabrication accuracy | Crack evaluation |
| | 1 | 3DP1 | 1.03 | B |
| Compara tive Example | 7 | 3DP2 | 1.03 | B |
| | 8 | 3DP3 | 1.04 | B |
| | 9 | 3DP4 | 1.03 | B |
| | 10 | 3DP5 | 1.04 | B |

As shown in Table 12, in Examples 21 to 32 using the curable compositions for photofabrication prepared in Examples 1, 14, and 18, results of the crack evaluation of the three-dimensional photofabricated products were "A1" or "A0" regardless of the type of the 3D printer. On the other hand, as shown in Table 13, in Comparative Examples 7 to 10 using the curable composition for photofabrication prepared in Comparative Example 1, results of the crack evaluation of the three-dimensional photofabricated products were "B" regardless of the type of the 3D printer.

## Claims

1. A curable composition for three-dimensional photofabrication to be used as a liquid photocurable composition held in a vat in a vat photopolymerization process for producing a three-dimensional photofabricated product by irradiating a predetermined position of the liquid photocurable composition with activating light including light having a specific wavelength λ (nm) in an ultraviolet light range or a visible light range and selectively curing the liquid photocurable composition present at the position, the curable composition comprising:
100 parts by mass of a polymerizable monomer component (A); 40 parts by mass to 400 parts by mass of an inorganic filler (B) containing a single type or a plurality of types of inorganic particulate matters; 0.01 parts by mass to 5 parts by mass of a photopolymerization initiator (C) having a function of initiating photopolymerization by irradiation with the activating light; and 0.01 parts by mass to 2.5 parts by mass of an activating light absorbent (D) having a function of absorbing the activating light and having no photopolymerization initiation ability,
80% or more of all primary particles constituting the inorganic filler (B) being particles having a particle diameter of 0.05 µm to 5.0 µm in a particle size distribution of the inorganic filler (B) measured by a microscopy method using a scanning microscope,
10 mass% to 90 mass% of the inorganic filler (B) being contained as an organic-inorganic composite filler (E) containing particles formed of a composite material of the inorganic filler (B) and a resin (R),
when, among the inorganic filler (B), an inorganic filler not combined with the resin (R) is defined as a non-composite inorganic filler (B1) and an inorganic filler contained as the organic-inorganic composite filler (E) is defined as a composite inorganic filler (B2), 80% or more of all primary particles constituting each of the non-composite inorganic filler (B1) and the composite inorganic filler (B2) being particles having a particle diameter of 0.05 µm to 5.0 µm,
the polymerizable monomer component (A), the non-composite inorganic filler (B1), and the organic-inorganic composite filler (E) being such that a value of a light scattering index Sc (%) of a base composition is 10 (%) or less,
the base composition being a composition consisting of the polymerizable monomer component (A), the non-composite inorganic filler (B1), and the organic-inorganic composite filler (E) and having a composition ratio of the components same as a composition ratio of the curable composition for three-dimensional photofabrication,
the light scattering index Sc (%) being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength λ (nm), including light having a wavelength in a range of λ ± 50 (nm) as a main component, and having a maximum intensity in the range, $Sc = \left\{\left({I}_{70}+{I}_{75}+{I}_{80}\right)/\left({I}_{0}\times 3\right)\right\} \times 100$
wherein I₀, I₇₀, I₇₅, and I₈₀ indicate intensities of the transmitted light in directions of emission angles of 0°, 70°, 75°, and 80°, respectively,
a transmittance to the activating light measured for a sample having a thickness of 0.5 mm and containing the curable composition for three-dimensional photofabrication being 1% to 50%.

2. A method for producing the curable composition for three-dimensional photofabrication according to claim 1, comprising:
a mixing step of mixing the polymerizable monomer component (A), the non-composite inorganic filler (B1), the organic-inorganic composite filler (E), the photopolymerization initiator (C), and the activating light absorbent (D), wherein
when a refractive index with respect to a line D at 25°C and a refractive index with respect to the activating light at 25°C of the polymerizable monomer component (A) are defined as n_{(D)M} and n_{(AL)M}, respectively, a refractive index with respect to the line D at 25°C and a refractive index with respect to the activating light at 25°C of the inorganic particulate matters constituting the inorganic filler (B) are defined as n_{(D)F} and n_{(AL)F}, respectively, a refractive index with respect to the activating light at 25°C of the resin (R) contained in the organic-inorganic composite filler (E) is defined as n_{(AL)R}, and a composition consisting of the polymerizable monomer component (A), the non-composite inorganic filler (B1), and the organic-inorganic composite filler (E) and having a composition ratio of the components same as a composition ratio of the curable composition for three-dimensional photofabrication serving as a production target is used as a base composition,
in the mixing step, those found to satisfy all of the following conditions 1 to 6 are used as the polymerizable monomer component (A), the non-composite inorganic filler (B1), and the organic-inorganic composite filler (E),
condition 1: when a particle diameter of each primary particle constituting the inorganic filler (B) is defined as x (nm) and a circumference ratio is defined as π, a total number of particles having a particle diameter x (nm) within a range of 0.7λ/π to 4λ/π (nm) is 40% or more of the number of all primary particles constituting the inorganic filler (B) in a particle size distribution of the inorganic filler (B) measured by a microscopy method using a scanning microscope;
condition 2: when an inorganic particulate matter consisting of an aggregate of a single type of inorganic particles having the refractive index n_{(D)F} in a range of 1.500 to 1.550 is defined as a specific inorganic particulate matter (b1) and an inorganic particulate matter consisting of an aggregate of a single type of inorganic particles having a refractive index out of the range is defined as a non-specific inorganic particulate matter (b2), each of the non-composite inorganic filler (B1) and the composite inorganic filler (B2)
(1) contains a single type of the specific inorganic particulate matter (b1),
(2) contains a plurality of types of the specific inorganic particulate matters (b1), at least one of the plurality of types of specific inorganic particulate matters (b1) occupying 10 mass% or more of a total mass of each of the non-composite inorganic filler (B1) and the composite inorganic filler (B2), or
(3) contains 90 mass% or more and less than 100 mass% of a single type or a plurality of types of the specific inorganic particulate matters (b1) and more than 0 mass% and 10 mass% or less of a single type or a plurality of types of the non-specific inorganic particulate matters (b2), at least one of the single type or the plurality of types of specific inorganic particulate matters (b1) occupying 10 mass% or more of a total mass of each of the non-composite inorganic filler (B1) and the composite inorganic filler (B2);
condition 3: the refractive index n_{(D)M} is within a range of 1.490 to 1.550;
condition 4: when a refractive index at which a difference with the refractive index n_{(D)M} is largest among the refractive indexes n_{(D)F} of at least one of the inorganic particulate matters (b1) occupying 10 mass% or more of the total mass of each of the non-composite inorganic filler (B1) and the composite inorganic filler (B2) is defined as n_{(D)Fh}, an absolute value |n_{(D)Fh} - n_{(D)M}| of the difference between n_{(D)Fh} and n_{(D)M} is 0.035 or less;
condition 5: the refractive index n_{(AL)F} of at least one of the inorganic particulate matters (b1) occupying 10 mass% or more of the total mass of each of the non-composite inorganic filler (B1) and the composite inorganic filler (B2), the refractive index n_{(AL)M}, and the refractive index n_{(AL)R} satisfy all relations shown in the following formulae (i) to (iii), $- 0.015 < \left({n}_{\left(AL\right) F}-{n}_{\left(AL\right) M}\right) < 0.025$ (i), $- 0.015 < \left({n}_{\left(AL\right) R}-{n}_{\left(AL\right) M}\right) < 0.030$ (ii), and $- 0.020 < \left({n}_{\left(AL\right) F}-{n}_{\left(AL\right) R}\right) < 0.020$ (iii); and
condition 6: a value of a light scattering index Sc (%) is 10% or less, the light scattering index Sc (%) being determined by the following formula based on an intensity of transmitted light in a specific emission angle direction obtained by measurement using a variable angle spectrophotometer in which a sample having a thickness of 0.5 mm and containing the base composition is vertically irradiated with measurement light including light having the specific wavelength λ (nm), including light having a wavelength in a range of λ ± 50 (nm) as a main component, and having a maximum intensity in the range, $Sc = \left\{\left({I}_{70}+{I}_{75}+{I}_{80}\right)/\left({I}_{0}\times 3\right)\right\} \times 100$
wherein I₀, I₇₀, I₇₅, and I₈₀ indicate intensities of the transmitted light in directions of emission angles of 0°, 70°, 75°, and 80°, respectively.

3. A method for producing a three-dimensional photofabricated product, which is a method for producing a three-dimensional photofabricated product by irradiating a predetermined position of a liquid photocurable composition held in a vat with activating light including light having a specific wavelength λ (nm) in an ultraviolet light range or a visible light range and selectively curing the liquid photocurable composition present at the position, the method comprising:
a shaping step of digitizing and ordinating a height direction of a three-dimensional object based on three-dimensional shape data indicating a shape of the three-dimensional object, generating two-dimensional shape data indicating a cross-sectional shape of the three-dimensional object at each ordinated height, irradiating the liquid photocurable composition held in the vat with the activating light at a predetermined position determined in advance based on the two-dimensional shape data, thereby selectively primarily curing the liquid photocurable composition present at the position, forming a fabricated layer having the cross-sectional shape, and sequentially forming and stacking the fabricated layer having the cross-sectional shape at each height according to an order of the ordinating, to obtain a stack having a shape corresponding to the shape of the three-dimensional object;
a washing step of washing, with an organic solvent, the stack obtained in the shaping step; and
a secondary curing step of secondarily curing the stack washed in the washing step by performing additional activating light irradiation or a heating treatment, or both of the additional activating light irradiation and the heating treatment,
the curable composition for three-dimensional photofabrication according to claim 1 being used as the liquid photocurable composition.

4. A method for producing a dental restoration, comprising: producing the dental restoration by the method for producing a three-dimensional photofabricated product according to claim 3.
